(19) Europäisches Patentamt
European Patent Office
Office européen des brevets

(11) EP 3 374 363 B1

(12) **FASCICULE DE BREVET EUROPEEN**

(45) Date de publication et mention de la délivrance du brevet:
**01.04.2020 Bulletin 2020/14**

(21) Numéro de dépôt: **16793866.1**

(22) Date de dépôt: **08.11.2016**

(51) Int Cl.:
***C07F 5/02*** *(2006.01)* ***C07C 29/153*** *(2006.01)*
***C07C 29/159*** *(2006.01)* ***C07C 31/04*** *(2006.01)*

(86) Numéro de dépôt international:
**PCT/EP2016/077010**

(87) Numéro de publication internationale:
**WO 2017/081024 (18.05.2017 Gazette 2017/20)**

(54) **PROCÉDÉ DE PRÉPARATION DE MÉTHOXYBORANES ET DE PRODUCTION DE MÉTHANOL**

VERFAHREN ZUR HERSTELLUNG VON METHOXYBORANEN UND ZUR HERSTELLUNG VON METHANOL

METHOD FOR PREPARING METHOXYBORANES AND FOR PRODUCING METHANOL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.11.2015 FR 1560747**

(43) Date de publication de la demande:
**19.09.2018 Bulletin 2018/38**

(73) Titulaire: **Commissariat à l'Énergie Atomique et aux Énergies Alternatives**
**75015 Paris (FR)**

(72) Inventeurs:
- **CHAUVIER, Clément**
  **75015 Paris (FR)**
- **CANTAT, Thibault**
  **92130 Issy Les Moulineaux (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
- **MARC-ANDRÉ COURTEMANCHE ET AL: "Reducing CO 2 to Methanol Using Frustrated Lewis Pairs: On the Mechanism of Phosphine-Borane-Mediated Hydroboration of CO 2", JOURNAL OF THE AMERICAN CHEMICAL SOCIETY, vol. 136, no. 30, 30 juillet 2014 (2014-07-30), pages 10708-10717, XP055264859, US ISSN: 0002-7863, DOI: 10.1021/ja5047846**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de préparation de méthoxyboranes par dismutation de l'acide formique ou d'au moins un de ses dérivés ou d'un mélange d'acide formique et d'au moins un de ses dérivés, en présence d'un organoborane, et éventuellement d'une base organique ou inorganique.

L'invention concerne également un procédé de production de méthanol comprenant une étape de préparation d'un méthoxyborane selon le procédé de l'invention et une étape d'hydrolyse ou de protonolyse du méthoxyborane en méthanol.

L'invention concerne, en outre, l'utilisation de méthoxyboranes obtenus par le procédé de l'invention pour la production du méthanol, comme catalyseurs dans des réactions d'allylation, comme réactifs dans les réactions de couplage de Suzuki, comme réactif pour la chimie fine ou pour la chimie lourde.

Le méthanol produit par hydrolyse ou protonolyse des méthoxyboranes formés par le procédé d'invention, peut être utilisé comme carburant ou additif dans les moteurs à combustion, comme carburant dans les piles à combustibles à méthanol direct, comme moyen de stockage de l'hydrogène, comme réactif pour la chimie fine.

Le méthanol, $CH_3OH$ (MeOH), est aujourd'hui au cœur d'une stratégie énergétique en ce sens qu'il est un candidat de premier plan pour supplanter les carburants fossiles tels que l'essence, le diesel ou le gaz. En effet, le méthanol possède des indices d'octane élevés et peut ainsi être utilisé directement comme carburant dans les moteurs actuels. De plus, celui-ci peut être employé dans des piles à combustible à méthanol direct (Direct Methanol Fuel Cell ou DMFC en anglais) c'est-à-dire des piles à combustible où le méthanol est introduit tel quel comme carburant pour générer de l'électricité et permet ainsi d'éviter l'emploi d'hydrogène $H_2$ gazeux, caractéristique des piles à combustibles classiques. Un autre secteur majeur d'application pour le méthanol est la production de produits chimiques et notamment des oléfines par le procédé *MTO* (*Methanol To Olefins*) ou encore de l'acide acétique par carbonylation (procédés Monsanto et plus récemment Cativa).

Cependant, les applications susmentionnées reposent essentiellement sur l'utilisation de méthanol obtenu à partir de ressources fossiles carbonées. Plus précisément, la source principale de méthanol à l'heure actuelle est le méthane ($CH_4$) fossile, qui, par reformage, donne du gaz de synthèse ($CO + H_2$) et celui-ci est finalement converti en méthanol. Ces procédés de reformage émettent du $CO_2$, gaz dont le rôle dans le réchauffement climatique est largement documenté. En conséquence, il existe un besoin grandissant en des procédés plus propres pour la production de méthanol, et notamment des procédés qui permettent la réintégration du $CO_2$ dans le cycle énergétique.

Actuellement, deux pistes principales sont explorées pour répondre à cette exigence :

- La réduction hexa-électronique (à 6 électrons *i.e.* 3 hydrures) du $CO_2$ en méthanol par hydrogénation catalytique (Figure 1 - Équation (1)),
- L'électro-réduction hexa-électronique du $CO_2$ en méthanol (Figure 1 - Équation (2)).

Ces deux approches présentent plusieurs inconvénients, notamment: (i) une faible sélectivité, c'est-à-dire qu'il y a obtention d'un mélange de produits ne contenant qu'un seul carbone (*i.e.* l'acide formique, monoxyde de carbone, formaldéhyde...) en plus du méthanol; (ii) des rendements faradiques (de transferts électroniques) faibles du fait du grand nombre d'électrons à transférer efficacement dans une réduction hexa-électronique; (iii) l'utilisation de métaux de transitions comme catalyseurs, et notamment de métaux nobles tels que le ruthénium et l'iridium dont le prix, la toxicité et/ou l'abondance posent généralement problème.

1. Hydrogénation du $CO_2$ en méthanol

La réaction d'hydrogénation du $CO_2$ en méthanol (équation (1) ci-dessus) nécessite l'utilisation de catalyseurs qui permettent de faciliter la réaction. En effet, bien que la thermodynamique soit favorable (réaction exergonique, $\Delta G^0 = - 40{,}9\ kJ.mol^{-1} < 0$), les barrières énergétiques successives qui conduisent à la formation de 3 liaisons C-H sont conséquentes, et ne peuvent être passées dans des conditions acceptables de température et pression.

Le premier catalyseur capable d'effectuer la réduction du $CO_2$ en méthanol a été reporté par Sasaki *et coll.* en 1993 [K.-i. Tominaga, Y. Sasaki, M. Kawai, T. Watanabe, M. Saito, J. Chem. Soc., Chem. Commun. 1993, 629]. Ces derniers ont utilisé le complexe $Ru_3(CO)_{12}$ en présence d'un sel d'halogénure KI pour obtenir du méthanol comme produit majoritaire en mélange avec d'autres produits de réduction tels que le monoxyde de carbone CO ou le méthane. La réaction est conduite dans la NMP (N-méthyl-2-pyrrolidone) à 240°C pendant 3heures sous 80 atmosphère (81,06 bar) d'un mélange $CO_2$:$H_2$ (1:3).

Cette réaction a été remise au goût du jour en 2011 par le groupe de Sanford aux USA [C. A. Huff, M. S. Sanford, J. Am. Chem. Soc. 2011, 133, 18122-18125]. Ces derniers ont mis au point un système catalytique complexe constitué de 3 catalyseurs différents. Chacun des trois catalyseurs est capable d'effectuer une étape de réduction différente. L'hydrogénation du $CO_2$ en acide formique HCOOH est catalysé par le complexe de ruthénium

$(PMe_3)_4Ru(Cl)(OAc)$. L'acide formique produit dans cette étape est alors estérifié en présence du méthanol deutéré utilisé comme solvant et d'une quantité catalytique de triflate de scandium $Sc(OTf)_3$. Finalement, le 3$^{ème}$ complexe (PNN)Ru(CO)(H) dans lequel PNN signifie le 6-(di-tert-butylphosphinométhylene)-2-(N,N-diéthylaminométhyl)-1,6-dihydropyridine, permet l'hydrogénation du formiate de méthyle en méthanol. Pour assurer la compatibilité entre les trois catalyseurs et les différentes espèces en présence dans le milieu réactionnel, la réaction est conduite dans un système à double chambre à 135°C pendant 16 heures pour obtenir le méthanol avec des TONs de 2,5 maximum. Des pressions en $CO_2$ et $H_2$ de 10 et 30 bars respectivement doivent être maintenues pour obtenir des rendements en méthanol corrects.

Pour éviter l'utilisation de 3 catalyseurs métalliques pour effectuer une unique transformation, Leitner, Klankermayer *et coll.* ont développé un système catalytique basé sur l'utilisation du seul complexe de ruthénium (Triphos)Ru(TMM) (TMM = triméthylèneméthane, Triphos = 1,1,1-tris(diphénylphosphinométhyl)éthane) qui, en présence d'un additif acide tel que l'acide triflimide ($HNTf_2$), permet d'obtenir le méthanol avec des TON allant jusqu'à 221 [S. Wesselbaum, T. Vom Stein, J. Klankermayer, W. Leitner, Angew. Chem. Int Ed. Engl. 2012, 51, 7499-7502]. La réaction se déroule dans le THF à 140°C sous une pression totale de 80 bar (60 bar $H_2$, 20 bar $CO_2$).

À la lueur des difficultés rencontrées dans l'hydrogénation catalytique du $CO_2$ en méthanol (rendement faible, conditions dures, utilisation de métaux nobles), une autre stratégie a été envisagée pour obtenir le méthanol à partir du $CO_2$, à savoir, la fonctionnalisation du $CO_2$ avant d'effectuer les étapes de réduction. Il est en effet connu que la formation de carbamates ou de carbonates par réaction du $CO_2$ avec respectivement, une amine ou un alcool est un processus favorable énergétiquement. Ainsi, de nombreux groupes de recherche travaillent sur l'hydrogénation de ces dérivés du $CO_2$ plutôt que sur ce dernier directement. Des exemples de réactions d'hydrogénation de carbonates ou de carbamates pour obtenir le méthanol sont documentés dans des revues récentes [E. Alberico, M. Nielsen, Chem. Comm. 2015, 51, 6714-6725 ; Y.-N. Li, R. Ma, L.-N. He, Z.-F. Diao, Catal. Sci. Technol. 2014, 4, 1498-1512.].

Par ailleurs, il est à noter qu'outre les catalyseurs homogènes basés sur des métaux de transitions cités ci-dessus, des catalyseurs hétérogènes tels que Cu/ZnO sont capables d'effectuer la réaction d'hydrogénation du $CO_2$ en méthanol [A. Goeppert, M. Czaun, J. P. Jones, G. K. Surya Prakash, G. A. Olah, Chem. Soc. Rev. 2014, 43, 7995-8048]. Cependant, la catalyse en phase homogène est plus avantageuse car, elle signifie souvent gain de sélectivité et conditions opératoires plus douces.

En parallèle des systèmes catalytiques basés sur des métaux de transition pour l'hydrogénation du $CO_2$, de nombreuses recherches sont dirigées vers le développement de systèmes qui ne contiennent pas de métaux et qui sont également capables d'hydrogéner le $CO_2$. La faisabilité de telles réactions sans métaux a été rendue possible par l'émergence des paires de Lewis frustrées, c'est-à-dire des composés ou mélanges contenant un acide de Lewis et une base de Lewis qui, en raison de leur encombrement stérique, ne peuvent pas se combiner pour former un adduit. Il a, en effet, été démontré en 2006 que la combinaison d'un acide de Lewis et d'une base de Lewis encombrés évite la formation d'un adduit classique de Lewis (d'où le terme "frustré") et permet en conséquence la fission de petites molécules telles que l'hydrogène en un proton et un hydrure [D. W. Stephan, Org. Biomol. Chem. 2008, 6, 1535-1539 ; G. C. Welch, R. R. San Juan, J. D. Masuda, D. W. Stephan, Science 2006, 314, 1124-1126].

Ce concept a été mis à profit en 2009 par O'Hare et Ashley qui ont montré pour la première fois qu'un système basé sur la combinaison du borane $B(C_6F_5)_3$ et de la base tétraméthylpipéridine (TMP) permet la réduction du $CO_2$ en méthanol en présence d'hydrogène $H_2$. Plus précisément, la paire de Lewis clive $H_2$ pour donner le borohydrure $[TMPH]^+[HB(C_6F_5)_3]^-$ qui réagit avec le $CO_2$ pour donner $[TMPH]^+[CH_3OB(C_6F_5)_3]^-$ qui est un précurseur du méthanol avant hydrolyse. Cette réaction s'effectue dans le toluène à 160°C pendant 6 jours sous des pressions de $CO_2$ et $H_2$ allant de 1 à 2 atm pour donner des rendements en méthanol de 25 % maximum [A. E. Ashley, A. L. Thompson, D. O'Hare, Angew. Chem. Int. Ed. Engl. 2009, 48, 9839-9843].

Plus récemment, une approche similaire a été reportée par Stephan, Fontaine *et coll.* [M. A. Courtemanche, A. P. Pulis, E. Rochette, M. A. Legare, D. W. Stephan, F. G. Fontaine, Chem. Comm. 2015, 51, 9797-9800] en utilisant la paire de Lewis frustrée intramoléculaire $1\text{-}BR_2\text{-}2\text{-}NMe_2\text{-}C_6H_4$ représentée ci-dessous en présence de $H_2$ et de $CO_2$. Néanmoins, les rendements en méthanol sont très faibles et un mélange de produits contenant un atome de carbone (*i.e.* formiates, acétals..) est obtenu.

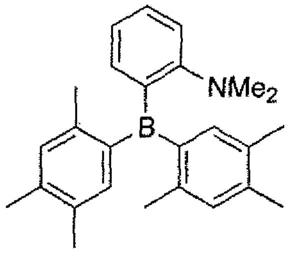

2. Électro- et photo-réductions hexa-électronique du $CO_2$ en méthanol

Deux difficultés majeures sont rencontrées lors de l'électro-réduction hexa-électronique du $CO_2$ en méthanol, à savoir : un problème de sélectivité pour le méthanol, c'est-à-dire l'obtention exclusive de ce dernier et pas d'autres produits contenant un atome de carbone tels que le CO ou l'acide formique. De plus, à la vue des potentiels redox, la réduction sélective du $CO_2$ en présence de protons est difficile car ces derniers peuvent aussi être réduits en hydrogène à des potentiels proches de ceux de l'électro-réduction. Ainsi, des électrocatalyseurs doivent être développés à la fois pour outrepasser les surtensions cinétiques importantes liées au transfert de 6 électrons, mais aussi pour assurer la sélectivité de la réduction.

De ce fait, il n'existe que très peu voire pas de systèmes capables d'effectuer efficacement l'électro-réduction à 6e du $CO_2$ en méthanol avec de bonnes sélectivités. A ce titre, les travaux de Bocarsly *et coll.* [G. Seshadri, C. Lin, A. B. Bocarsly, J. Electroanal. Chem. 1994, 372, 145-150] ont montré dès 1994 que le cation pyridinium $PyH^+$ permet de catalyser en phase homogène l'électro-réduction sur électrode de Pd du $CO_2$ en méthanol avec des rendements faradiques atteignant 30%. L'intérêt de ce système étant que les surtensions appliquées sont relativement faibles comparés à de nombreux autres systèmes. D'autres exemples peuvent être trouvés dans des revues récentes telles que celles de Olah[A. Goeppert, M. Czaun, J. P. Jones, G. K. Surya Prakash, G. A. Olah, Chem. Soc. Rev. 2014, 43, 7995-8048] ou de Kenis [H.-R. M. Jhong, S. Ma, P. J. A. Kenis, Curr. Opin. Chem. Eng. 2013, 2, 191-199].

3. Hydrogénation et électro-réduction à 2 électrons du $CO_2$ en acide formique

• Hydrogénation à 2 électrons du $CO_2$ en HCOOH

Cette réaction est thermodynamiquement défavorisée ($\Delta G^0$ = +32,9 kJ.mol$^{-1}$), et l'addition d'une base est nécessaire pour déplacer l'équilibre vers la formation du sel $HCOO^-$, $BaseH^+$.

Le catalyseur le plus actif à ce jour pour cette transformation est un complexe d'iridium PNP-$IrH_3$ (PNP = 2,6-bis(di-tert-butylphosphinométhyl)pyridine) rapporté par Nozaki *et coll.* en 2009[R. Tanaka, M. Yamashita, K. Nozaki, J. Am. Chem. Soc. 2009, 131, 14168-14169]. Des TONs de 3500000 et des TOFs de 150000 $h^{-1}$ ont été obtenus en solution aqueuse de potasse KOH à 120°C après 48h de réaction sous une pression totale de 60 bar (30/30 $CO_2/H_2$). Le produit de réaction est alors le formiate de potassium HCOOK, qui en présence d'un acide fort peut libérer l'acide formique HCOOH [C. Federsel, R. Jackstell, M. Beller, Angew. Chem. Int. Ed. Engl. 2010, 49, 6254-6257].

En 2012, Beller *et coll.* [C. Ziebart, C. Federsel, P. Anbarasan, R. Jackstell, W. Baumann, A. Spannenberg, M. Beller, J. Am. Chem. Soc. 2012, 134, 20701-20704] ont montré que le complexe de fer $Fe(BF_4)_2{\cdot}6H_2O$ mis en présence du ligand tris(2-(diphénylphosphino)phényl)phosphine permet l'hydrogénation du $CO_2$ en acide formique et en produits dérivés de tels que le diméthylformamide (DMF) lorsque la diméthylamine est ajoutée au milieu réactionnel ou le formiate de méthyle lorsque le méthanol est solvant. Des TONs d'environ 2000 sont obtenus à 100°C sous des pressions totales de 60 bars (30/30 $CO_2/H_2$).

Dyson *et coll.* [S. Moret, P. J. Dyson, G. Laurenczy, Nat. Commun. 2014, 5, 4017] décrit un catalyseur à base de ruthénium qui permet d'effectuer la réaction d'hydrogénation du $CO_2$ en solution aqueuse sans additif basique (mentionnée plus haut comme étant nécessaire pour déplacer l'équilibre). Sous une pression totale de 100 bar (50/50 $CO_2/H_2$), le complexe $RuCl_2(PTA)_4$ (PTA = 1,3,5-triaza-7-phosphaadamantane) effectue jusqu'à 159 TONs et l'acide formique HCOOH est obtenu à une concentration de 0,1 M dans l'eau. Si le DMSO est utilisé comme solvant, des TONs de 475 peuvent être atteint.

• Électro-réduction à 2 $e^-$ du $CO_2$ en HCOOH

Cette stratégie est aujourd'hui la seule voie électrochimique de réduction du $CO_2$ économiquement viable. En effet, l'acide formique et les formiates correspondant sont aujourd'hui les seuls produits contenant un atome de carbone à pouvoir être généré avec un impact énergétique relativement faible et avec des rendements faradiques importants. Par ailleurs, le marché acide formique a de bonne chance de s'accroitre dramatiquement du fait du grand nombre d'application de l'acide formique, notamment comme candidat pour stocker l'hydrogène pour des applications légères, c'est-à-dire des applications avec une demande énergétique limitée comme la téléphonie mobile [A. S. Agarwal, Y. Zhai, D. Hill, N. Sridhar, ChemSusChem 2011, 4, 1301-1310 ; X. Lu, D. Y. C. Leung, H. Wang, M. K. H. Leung, J. Xuan, ChemElectroChem 2014, 1, 836-849]. Il est à noter que la production actuelle d'acide formique est évaluée à 800 kT.an$^{-1}$.

De nombreux systèmes basés sur des catalyseurs homogènes ou hétérogènes sont viables pour l'électro-réduction à 2 électrons du $CO_2$ en acide formique, c'est-à-dire que les rendements faradiques obtenus sont supérieurs à 80 % avec des densités de courant relativement grandes. Par exemple, lorsque l'électrode de travail est recouverte d'une couche de Pd, des rendements faradiques de 100 % sont obtenus [B. I. Podlovchenko, E. A. Kolyadko, S. Lu, J. Electroanal. Chem. 1994, 373, 185-187]. De nombreux exemples ainsi qu'une analyse technico-économique de la

viabilité du procédé $CO_2$ vers HCOOH ont été publiés.

4. Dismutation de l'acide formique en méthanol et $CO_2$

De tout ce qui précède, il apparaît que la formation de méthanol directement à partir du $CO_2$ par réduction hexa-électronique (par hydrogénation ou électro-réduction) n'est pas favorable et encore moins économiquement viable. A contrario, l'électroréduction à 2 $e^-$ est en passe de le devenir.

Une alternative aux procédés précédents se base sur la dismutation de l'acide formique en $CO_2$ et méthanol comme montré en Figure 2 (Equation 3). Dans ce procédé, l'acide formique peut provenir de l'hydrogénation ou plus avantageusement de l'électro-réduction à $2e^-$ du $CO_2$. Le $CO_2$ produit par la réaction de dismutation peut être reconverti en acide formique par le même procédé.

La dismutation est une réaction dans laquelle une espèce chimique, en l'occurrence l'acide formique, joue à la fois le rôle d'oxydant et de réducteur. Cela signifie que l'espèce chimique, initialement présente à un seul degré d'oxydation, se trouvera, après la réaction, sous forme de deux espèces, l'une oxydée, l'autre réduite.

Dans le cas présent, la réaction de dismutation permet d'utiliser l'acide formique, qui est liquide à température et pression ordinaire, à la fois comme source de carbone pour des produits contenant un atome de carbone mais aussi comme source d'hydrure pour assurer l'obtention de méthanol.

Il est à noter en premier lieu que la réaction de dismutation de l'acide formique en méthanol et $CO_2$ est en compétition avec deux autres réactions plus classiques de décomposition de l'acide formique montrées en Figure 3 et que sont :

- la déshydrogénation de l'acide formique en $CO_2$ et $H_2$ (Équation (4))
- la déshydratation de l'acide formique en CO et $H_2O$ (Équation (5))

L'état de l'art actuel des catalyseurs capables d'effectuer la réaction de dismutation (3) plutôt que (4) et/ou (5) sont en nombre très limités. Ces derniers opèrent exclusivement en phase homogène et sont tous basés sur des complexes de métaux de transition.

Les premiers travaux dans le domaine ont été réalisés en 2013 par Miller, Goldberg *et coll.* [A. J. Miller, D. M. Heinekey, J. M. Mayer, K. I. Goldberg, Angew. Chem. Int. Ed. Engl. 2013, 52, 3981-3984]. Les auteurs ont montré que le complexe d'iridium $[Cp^*Ir(bpy)(H_2O)](OTf)_2$ ($Cp^*$ = pentaméthylcyclopentadienyl, bpy = 2,2'-bipyridine) permet d'obtenir des rendements en méthanol allant jusqu'à 12 %. Ce chiffre représente la sélectivité de la réaction pour la formation de méthanol selon l'équation (3). Cela signifie encore que 88 % des liaisons C-H dans H-COOH terminent en hydrogène selon l'équation (4) et non en méthanol. Ces 12 % de sélectivité sont obtenus à 60°C en présence d'acide formique en solution aqueuse (C = 12 M). La conversion de l'acide formique dans ce cas n'est que de 3 %.

Plus récemment, Cantat *et al.* [S. Savourey, G. Lefevre, J. C. Berthet, P. Thuery, C. Genre, T. Cantat, Angew. Chem. Int. Ed. Engl. 2014, 53, 10466-10470] ont montré qu'en utilisant un complexe de ruthénium $[Ru(COD)(méthylallyl)_2]$ (COD = cyclooctadiène) combiné avec le ligand tridente "triphos" $CH_3C(CH_2PPh_2)_3$ dans le THF à 150°C, la sélectivité (qui correspond ici au rendement en méthanol) peut atteindre 26,7 % . Dans les mêmes conditions et en présence d'un additif acide (acide méthane sulfonique ou MSA), le rendement dépasse les 50 %. Ce dernier résultat représente à l'heure actuelle le rendement le plus important en méthanol obtenu par dismutation de l'acide formique.

En dernier lieu, Parkin *et al.* ont démontré en 2015 [M. C. Neary, G. Parkin, Chem. Sci. 2015, 6, 1859-1865] que des composés de molybdène sont capables d'effectuer la réaction de dismutation de l'acide formique. Plus précisément, le complexe $CpMo(CO)_3H$ (Cp = cyclopentadienyle) est le plus efficace des complexes évalués et une sélectivité de 21 % est reportée à 100°C dans le benzène. Le procède décrit dans le schéma 8 de MARC-ANDRE COURTEMANCHE ET AL: "Reducing CO 2 to Methanol Using Frustrated Lewis Pairs: On the Mechanism of Phosphine-Borane-Mediated Hydroboration of CO 2",JOURNAL OF THE AMERICAN CHEMICAL SOCIETY, vol. 136, no. 30, 2014 et le texte s'y rapportant montrent clairement que le méthoxyborane est obtenu par la réduction de l'acide formique avec un excès (3,3 équivalents) de CatBH (catéchol borane) (conditions d'hydroboration) et non par dismutation du formiate dans les conditions indiquées dans la revendication 1 de la présente demande.

Cet état de l'art révèle que la réaction de dismutation de l'acide formique est, à l'heure actuelle, exclusivement rendue possible par l'emploi de métaux, le plus souvent nobles et peu sélectifs. Il n'existe pas à l'heure actuelle de composés non-métalliques pouvant permettre d'effectuer cette transformation.

Il existe donc un réel besoin d'un composé non-métallique permettant la dismutation sans métal de l'acide formique.

En particulier, il existe un réel besoin d'un composé qui permet la dismutation sans métal de l'acide formique, et qui ne contient pas :

- de métaux alcalinoterreux du Groupe IIA du Tableau Périodique des Eléments (comme le magnésium et le calcium) ;

- de métaux de transition du Groupe IB à VIIIB du Tableau Périodique des Eléments (comme le nickel, le fer, le cobalt, le zinc, le cuivre, le rhodium, le ruthénium, le platine, le palladium, l'iridium) ;
- de terres rares dont le numéro atomique est compris entre 57 et 71 (comme le lanthane, le cérium, le praséodyme, le néodyme) ; ou
- d'actinides dont le numéro atomique est compris entre 89 et 103 (comme le thorium, l'uranium).

Il existe également un réel besoin d'un composé non-métallique tel que défini ci-dessus, permettant la dismutation de l'acide formique en méthanol, qui soit efficace (capable d'augmenter la vitesse de la conversion de l'acide formique en méthanol même lorsqu'il est présent en faible quantité), sélectif (favorisant la production du produit désiré par rapport aux produits secondaires), peu coûteux et/ou peu toxique comparé aux complexes métalliques connus pour la réaction dismutation de l'acide formique en méthanol.

La présente invention a précisément pour but de répondre à ces besoins en fournissant un procédé de préparation de méthoxyboranes de formule (I)

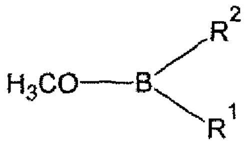

(I)

dans laquelle

- $R^1$ et $R^2$, indépendamment l'un de l'autre, sont choisis dans le groupe formé par un groupe hydroxyle, un groupe alcoxy, un groupe alkyle, un groupe alcényle, un groupe alcynyle, un groupe aryle, un groupe hétéroaryle, un groupe hétérocyclique, un atome d'halogène, un groupe silylé, un groupe siloxy, un groupe phosphino, et un groupe amino, lesdits groupes alkyle, alcényle, alcynyle, alcoxy, silyle, siloxy, aryle, hétéroaryle, hétérocyclique, phosphino et amino étant éventuellement substitués ; ou
- $R^1$ et $R^2$ pris ensemble avec l'atome de bore auquel ils sont liés, forment un hétérocycle éventuellement substitué ;

par dismutation de l'acide formique ou d'au moins un de ses dérivés de formule $HCO_2M$ dans laquelle M est choisi dans le groupe formé par $Na^+$, $K^+$, $Li^+$, $Cs^+$, $NH_4^+$, le triéthylammonium ($HNEt_3^+$), le tétraphénylphosphonium ($PPh_4^+$), le tétraméthylammonium ($NMe_4^+$), le tétraéthylammonium ($NEt_4^+$), le tétrabutylammonium ($NBu_4^+$) et le tétraphénylammonium ($NPh_4^+$), ou d'un mélange d'acide formique et d'au moins un de ses dérivés tel que défini ci-dessus,
en présence

- d'un organoborane de formule (II) dans laquelle

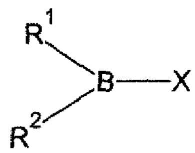

(II)

  - $R^1$ et $R^2$ sont tels que définis pour les composés méthoxyborane de formule (I) ;
  - X est choisi dans le groupe formé par un atome d'hydrogène, un atome d'halogène, un groupe carboxylate, un groupe sulfonate, un groupe hydroxyle, un groupe alcoxy, un groupe alkyle, un groupe alcényle, un groupe alcynyle, un groupe aryle, un groupe hétéroaryle, un groupe hétérocyclique, un groupe silylé, un groupe siloxy, un groupe phosphino, un groupe amino, lesdits groupes alkyle, alcényle, alcynyle, alcoxy, silyle, siloxy, aryle, hétéroaryle, hétérocyclique, phosphino et amino étant éventuellement substitués ; et éventuellement

- d'une base organique.

L'organoborane de formule (II) permet ainsi la dismutation sans métal de l'acide formique ou de l'un de ses dérivés.

Le méthoxy borane de formule (I) peut ensuite être soumis à une hydrolyse ou à une protonolyse pour fournir du méthanol.

L'étape d'hydrolyse ou de protonolyse permet de transformer le méthoxyborane de formule (I) en méthanol

libre $CH_3OH$, éventuellement en régénérant les organoboranes de formule (II).

En fait, une fois les méthoxyboranes obtenus, deux possibilités sont envisageables pour former le méthanol libre : par simple réaction avec l'eau (hydrolyse) ou bien avec un acide plus fort (protonolyse) comme par exemple l'acide formique, l'acide acétique ou l'acide chlorhydrique.

Les organoboranes de formule (II) obtenus après l'étape de protonolyse ou d'hydrolyse des méthoxyboranes peuvent être réutilisés pour produire les méthoxyboranes par le procédé d'invention.

Le procédé de production de méthanol mettant en jeu une étape de dismutation de l'acide formique, de l'un de ses dérivés, ou de leur mélange comme définis ci-dessus, possède les avantages suivants :

- L'acide formique est liquide dans les conditions normales de température (20 ± 5°C) et pression (1 atmosphère ou 1,01325 bar), ce qui le rend facile à transporter avec des coûts (et donc des risques) plus faibles que le transport de gaz tel que l'hydrogène. Cela est particulièrement avantageux lorsque la production de l'acide formique et la dismutation sont effectués dans deux lieux distincts. Cette étape revient donc à stocker l'hydrogène et le $CO_2$ sous forme d'acide formique.
- L'acide formique est non toxique en solution diluée (concentration inférieure à 85% massique dans l'eau) et est de fait un réactif bénin.
- Dans le présent procédé, la réaction de dismutation est effectuée à l'aide de dérivés organiques du bore donc sans métal, ce qui évite les inconvénients des dérivés métalliques existants, notamment un coût et une toxicité problématiques.
- Les organoboranes utilisés de formule (II) sont commerciaux, ou facilement préparés par l'homme du métier à partir de réactifs commerciaux et bons marchés. A contrario, lorsque des métaux sont utilisés, les ligands employés pour les stabiliser sont chers et leur préparation nécessite le plus souvent de multiples étapes de synthèse.

Au sens de la présente invention un groupe « alkyle » désigne un radical carboné linéaire, ramifié ou cyclique, saturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cyclique, on peut citer les radicaux cyclopropyle, cyclobutyle, cyclopentyle, cyclohexyle, cycloheptyle, cyclooctyle, bicylco[2,1,1] hexyle, bicyclo[2,2,1] heptyle. Le groupe alkyle peut comprendre par exemple 1 à 8 atomes de carbone.

Par « alcényle » ou « alcynyle », on entend un radical carboné insaturé linéaire, ramifié ou cyclique, éventuellement substitué, ledit radical carboné insaturé comprenant 2 à 12 atomes de carbone comprenant au moins une double (alcényle) ou une triple liaison (alcynyle). A ce titre, on peut citer, par exemple, les radicaux éthylényle, propylényle, butényle, pentényle, hexényle, acétylényle, propynyle, butynyle, pentynyle, hexynyle et leurs isomères ramifiés. Comme alcényle cycliques, on peut citer par exemple le cyclopentényle, le cyclohexényle. Les groupes alcényle et alcynyle peuvent comprendre par exemple 2 à 8 atomes de carbone.

Les groupes alkyle, alcényle, alcynyle peuvent être éventuellement substitués par un ou plusieurs groupes hydroxyle ; un ou plusieurs groupes alkoxy ; un ou plusieurs atomes d'halogène choisis parmi les atomes de fluor, chlore, brome ou iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs groupes siloxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkoxy, alkyle et siloxy tels que définis dans le cadre de la présente invention. Le groupe aryle peut comprendre par exemple, 6 à 10 atomes de carbone.

Le terme « hétéroaryle » désigne de manière générale un substituant aromatique mono- ou polycyclique comportant de 5 à 12 membres dont au moins 2 atomes de carbone, et au moins un hétéroatome choisi parmi l'azote, l'oxygène, le bore, le silicium, le phosphore ou le soufre. Le groupe hétéroaryle peut être mono- ou poly-cyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme «hétérocycle ou hétérocyclique » désigne de manière générale un substituant mono- ou polycyclique, comportant de 5 à 10 membres, saturé ou insaturé, contenant de 1 à 4 hétéroatomes choisis indépendamment l'un de

l'autre, parmi l'azote, l'oxygène, le bore, le silicium, le phosphore ou le soufre. A titre indicatif, on peut citer le borolane, le borole, le borinane, le 9-borabicyclo[3.3.1]nonane (9-BBN), le 1,3,2-benzodioxaborole (catecholborane ou catBH), pinacholborane (pinBH), les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Le terme « alcoxy » signifie un groupe alkyle, tels que défini ci-dessus, lié par un atome d'oxygène (-O-alkyle).

Par groupe « amino », on entend un groupe de formule $-NR^3R^4$, dans laquelle :

- $R^3$ et $R^4$ représentent, indépendamment l'un de l'autre, un groupe alkyle, un groupe alcényle, un groupe alcynyle, un groupe aryle, un groupe hétéroaryle, un groupe hétérocyclique, un groupe silyle, un groupe siloxy, avec les groupes alkyle, alcényle, alcynyle, aryle, hétéroaryle, hétérocyclique, silyle, siloxy, tels que définis dans le cadre de la présente invention ; ou
- $R^3$ et $R^4$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Le groupe amino peut être, par exemple, un substituant choisi dans le groupe formé par $-NEt_2$, pipéridinyle, morpholinyle, méthylaniline ($C_6H_5N(CH_3)$-), et triazabicyclodécényle (TBD).

Par groupe « phosphino », on entend un groupe de formule $-PR^5R^6$, dans laquelle :

- $R^5$ et $R^6$ représentent, indépendamment l'un de l'autre, un groupe alkyle, un groupe alcényle, un groupe alcynyle, un groupe aryle, un groupe hétéroaryle, un groupe hétérocyclique, un groupe silyle, un groupe siloxy, avec les groupes alkyle, alcényle, alcynyle, aryle, hétéroaryle, hétérocyclique, silyle, siloxy, tels que définis dans le cadre de la présente invention ; ou
- $R^5$ et $R^6$, pris ensemble avec l'atome de phosphore auquel ils sont liés, forment un hétérocycle éventuellement substitué par un ou plusieurs groupes hydroxyle; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Le groupe phosphino peut être, par exemple, un substituant choisi dans le groupe formé par $PCy_2$ avec Cy=cyclohexyle, et $PPh_2$ avec Ph=phényle.

Par atome d'halogène, on entend un atome choisi parmi les atomes de fluor, chlore, brome et iode.

Le terme « sulfonate » désigne un groupe de formule $-OSO_2R^7$, dans lequel :

- $R^7$ est choisi parmi : un groupe méthyle ($CH_3$), un groupe trifluorométhyle ($CF_3$), un groupe toluène ($p$-$CH_3C_6H_4$) ou un groupe benzène ($C_6H_5$).

Le terme « carboxylate » désigne un groupe de formule $-OC(O)R^8$, dans lequel $R^8$ est choisi parmi un atome d'hydrogène , un groupe alkyle, un groupe alcényle, un groupe alcynyle, un groupe aryle, un groupe hétéroaryle, un groupe hétérocyclique, un groupe silyle, un groupe siloxy, avec les groupes alkyle, alcényle, alcynyle, aryle, hétéroaryle, hétérocyclique, tels que définis dans le cadre de la présente invention.

Par groupe « silyle », on entend un groupe de formule $[-Si(Y)_3]$ dans lequel chaque Y, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes amino ; un ou plusieurs groupes aryle ; un ou plusieurs groupes siloxy ; avec les groupes alkyle, alkoxy, aryle et siloxy tels que définis dans le cadre de la présente invention. A ce titre on peut citer, par exemple, le triméthylsilyle (TMS), le triéthylsilyle (TES), le *tert*-butyldiphénylsilyle (TBDPS), le *tert*-butyldiméthylsilyle (TBS/TBDMS), le triisopropylsilyle (TIPS), le tri(triméthylsilyl)silyle ou $((CH_3)_3Si)_3Si$- (TTMS), le tri(*tert*-butyl)silyle ou $((CH_3)_3C)_3Si$-.

Par groupe « siloxy », on entend un groupe silyle, tel que défini ci-dessus, lié par un atome d'oxygène ($-O-Si(Y)_3$) avec Y tel que défini ci-dessus. A ce titre on peut citer, par exemple, le triméthylsiloxy, le triéthylsiloxy, le *tert*-butyldiphénylsiloxy.

Dans le contexte de l'invention, la « protonolyse » signifie le clivage d'une liaison chimique par des acides de Bronsted. L'hydrolyse signifie le clivage d'une liaison chimique par l'eau.

Selon une première variante de l'invention, dans le méthoxyborane de formule (I) et l'organoborane de formule (II), $R^1$ et $R^2$, indépendamment l'un de l'autre, sont choisis dans le groupe formé par un groupe alkyle comprenant 1 à 12, de préférence 1 à 8 atomes de carbone ; un aryle comprenant 6 à 20, de préférence 6 à 10 atomes de carbone,

lesdits groupes alkyle et aryles étant éventuellement substitués. De préférence, $R^1$ et $R^2$, indépendamment l'un de l'autre, sont choisis dans le groupe formé par méthyle, éthyle, propyle, butyle, pentyle, hexyle, heptyle et octyle et leurs isomères ramifiés, cyclohexyle, phényle, benzyle. Encore plus préférentiellement, $R^1$ et $R^2$, indépendamment l'un de l'autre, sont choisis dans le groupe formé par butyle et ses isomères ramifiés, et cyclohexyle.

Selon une deuxième variante de l'invention, dans le méthoxyborane de formule (I) et l'oganoborane de formule (II), $R^1$ et $R^2$ pris ensemble avec l'atome de bore auquel ils sont liés, forment un hétérocycle comportant 5 à 10 membres, ledit hétérocycle étant éventuellement substitué. De préférence, $R^1$ et $R^2$ pris ensemble avec l'atome de bore auquel ils sont liés, forment un 9-borabicyclo[3.3.1]nonane (9-BBN), un 1,3,2-benzodioxaborole (catecholborane ou catBH), ou un pinacholborane (pinBH).

Dans toutes les variantes et tous les modes préférés, X peut par exemple être choisi dans le groupe formé par un atome d'hydrogène ; un groupe carboxylate de formule $-OCOR^8$ dans laquelle $R^8$ est choisi parmi un atome d'hydrogène, un groupe alkyle comprenant 1 à 12, de préférence 1 à 8 atomes de carbone ; un atome d'halogène ; un groupe alkyle comprenant 1 à 12, de préférence 1 à 8 atomes de carbone ; un groupe sulfonate de formule $-OSO_2R^7$, dans lequel $R^7$ est choisi parmi un groupe méthyle ($CH_3$), un groupe trifluorométhyle ($CF_3$), un groupe toluène ($p$-$CH_3C_6H_4$) ou un groupe benzène ($C_6H_5$). En particulier, X est choisi dans le groupe formé par un atome d'hydrogène ; un groupe carboxylate de formule $-OCOR^8$ dans laquelle $R^8$=H; un atome de chlore ; un atome de bore ; un groupe alkyle choisi dans le groupe formé par méthyle, éthyle, propyle, butyle, pentyle, hexyle et leurs isomères ramifiés ; un groupe sulfonate de formule-$OSO_2R^7$, dans lequel $R^7$ est choisi parmi un méthyle un groupe trifluorométhyle ($CF_3$), un groupe toluène ($p$-$CH_3C_6H_4$) ou un groupe benzène ($C_6H_5$).

De préférence, l'organoborane de formule (II) peut être choisi dans le groupe formé par le tributylborane, le dicyclohexylborane, l'iododicyclohexylborane, le dibutylborane méthanesulfonate ($n$-$Bu_2BOSO_3Me$), le B-iodo-9-borabicyclo[3.3.1]nonane, le dimère de 9-borabicyclo[3.3.1]nonane, le B-benzyl-9-borabicyclo[3.3.1]nonane, le Me-TBD-$BBN^+I^-$.

La dismutation de l'acide formique, d'au moins un de ses dérivés, ou de leur mélange tel que décrit précédemment, peut avoir lieu avantageusement en présence d'une base organique ou inorganique, de préférence organique.

La base organique peut être choisie parmi :

- les bases organiques azotées qui sont avantageusement des amines tertiaires choisies dans le groupe formé par, par exemple, la triéthylamine, la triméthylamine, la *N*-diisopropyléthylamine (DIPEA), la diéthylisopropylamine (DIEA), la 7-Méthyl-1,5,7-triazabicyclo[4.4.0]dec-5-ène (MTBD), le 1,8-diazabicyclo[5.4.0]undéc-7-ène (DBU), le 1,4-diazabicyclo[2.2.2]octane (DABCO), et la *N*-méthylpiperidine ;
- les bases organiques phosphorées pouvant être des alkyle ou aryles phosphines choisies dans le groupe formé par, par exemple, la triphénylphosphine, le 2,2'-bise(diphénylphosphino)-1,1'-binaphthyle (BINAP), le triisopropylphosphine, 1,2-bis(diphénylphosphino)éthane (dppe), la tricyclohexylphosphine ($PCy_3$) ; les aza-phosphines choisies dans le groupe formé par, par exemple, le 2,8,9-triisopropyl-2,5,8,9-tétraaza-1-phosphabicyclo[3.3.3]undecane ($BV^{Me}$) et le 2,8,9-triisobutyl-2,5,8,9-tétraaza-1-phosphabicyclo[3.3.3]undecane ($BV^{iBu}$) ;
- les bases carbonées pour lesquelles la protonation a lieu sur un atome de carbone, choisies avantageusement parmi les carbènes N-hétérocycliques issus d'un sel d'imidazolium, ledits carbènes étant, par exemple, choisis dans le groupe formé par les sels de 1,3-bis(2,6-diisopropylphényl)-1H-imidazol-3-ium (également appelé IPr), 1,3-bis(2,6-diisopropylphényl)-4,5-dihydro-1H-imidazol-3-ium (également appelé s-IPr), 1,3-bis(2,4,6-triméthylphényl)-1H-imidazol-3-ium (également appelé IMes), 1,3-bis(2,4,6-triméthylphényl)-4,5-dihydro-1H-imidazol-3-ium (également s-IMes), 4,5-dichloro-1,3-bis(2,6-diisopropylphényl)-1H-imidazol-3-ium (également appelé $Cl_2$-IPr), 1,3-di-tert-butyl-1H-imidazol-3-ium (également appelé ItBu), et 1,3-di-tert-butyl-4,5-dihydro-1H-imidazol-3-ium (également appelé s-ItBu), lesdits sels étant sous la forme de sels de chlorure ou de tétraphénylborate, par exemple.

Des exemples de carbènes N-hétérocycliques sont représentés dans la Figure 4.

De préférence, la base organique est une base organique azotée choisie dans le groupe formé par la triéthylamine, la triméthylamine, la *N*-diisopropyléthylamine (DIPEA), la diéthylisopropylamine (DIEA), la 7-Méthyl-1,5,7-triazabicyclo[4.4.0]dec-5-ène (MTBD), le 1,8-diazabicyclo[5.4.0]undéc-7-ène (DBU), le 1,4-diazabicyclo[2.2.2]octane (DABCO), et la *N*-méthylpiperidine.

Lorsqu'on part du dérivé de l'acide formique de formule HCOOM (par exemple HCOONa), et qu'on le fait réagir sur l'organoborane de formule (II), il n'est en général pas nécessaire d'introduire une base dans le milieu car l'acide formique a déjà été déprotonné pour obtenir ce dérivé. Il en va de même si X=H et que l'on utilise un mélange d'acide formique pur et de HCOONa (1:1), par exemple.

Lorsque cela s'avère nécessaire, un additif peut également être utilisé pour solubiliser les réactifs de départ. Par additif, au sens de l'invention, on entend tout composé capable d'améliorer la solubilité des réactifs intervenant dans la dismutation de l'acide formique, de l'un de ses dérivés ou d'un mélange d'acide formique et d'au moins un de ses dérivés. Ces additifs sont introduits lorsque des dérivés de l'acide formique tels que les sels alcalins de l'anion formiate

sont employés (par exemple HCOONa ou HCOOK) ou encore lorsqu'une base inorganique est employée. La dismutation de l'acide formique, ou d'au moins uns de ses dérivés ou d'un mélange d'acide formique et d'au moins un de ses dérivés comme définis précédemment, peut avoir lieu, en outre, en présence d'un additif. Ces additifs peuvent être choisis, par exemple, parmi :

- les éthers couronnes choisis dans le groupe formé par le 12-couronne-4, le 15-couronne-5, le 18-couronne-6, le dibenzo-18-couronne-6, le benzo-18-couronne-6, le benzo-15-couronne-5, et le dibenzo-15-couronne-5 ;
- les aza-couronnes choisis dans le groupe formé par le 1,4,7,10-tétraazacyclododécane (cyclène), le 1,4,7,10,13,16-hexaazacyclooctadécane (hexacyclène), et le diaza-18-couronne-6 ;
- les thioéthers couronnes choisis dans le groupe formé parle 1,5,9,13-tétrathiacyclohexadécane (16-Ane-$S_4$), et le 1,4,7,10,13,16-hexathiacyclooctadécane (18-Ane-$S_6$).

Les organoboranes de formule (II) peuvent, le cas échéant, être immobilisés sur des supports hétérogènes afin d'assurer leur séparation facile et/ou leur recyclage. Lesdits supports hétérogènes peuvent être choisis parmi les supports à base de gel de silice ou de polymères plastiques comme, par exemple, le polystyrène ; les supports carbonés choisis parmi les nanotubes de carbone ; le carbure de silice ; ou l'alumine.

L'acide formique ou l'un de ses dérivés comme définis précédemment peut être préparé par tout procédé connu de l'homme du métier. Dans le présent exposé, des procédés connus pour pouvoir réaliser cette étape ont été décrits. De préférence, l'acide formique est préparé par une électro-réduction à 2 $e^-$ ou une hydrogénation catalytique du $CO_2$ comme montré en Figure 5.

Les dérivés de l'acide formique sont généralement obtenus à partir de celui-ci. Cependant, il existe un grand nombre de procédés, et notamment l'hydrogénation du $CO_2$, qui permettent d'obtenir les dérivés de l'acide formique directement. L'obtention de l'acide formique protonné demande une étape supplémentaire, typiquement par addition d'un acide fort. Les références décrivant la préparation de dérivés de l'acide formique sont celles précités qui décrivent l'hydrogénation du $CO_2$. Les dérivés de l'acide formique peuvent également être préparés très facilement par tout procédé bien connu de l'homme du métier, à savoir, par la réaction d'une base (par exemple la triéthylamine) avec l'acide formique. Il est à noter que la base employé doit avoir un pKa supérieur à celui de l'acide formique, c'est-à-dire supérieur à 3,7

L'acide formique peut également être généré à partir de l'un de ses dérivés par réaction avec de l'acide chlorhydrique (reprotonnation du sel) ou tout autre acide fort dans l'eau.

Comme déjà indiqué, l'hydrogénation catalytique du $CO_2$ nécessite l'emploi d'une base pour déplacer l'équilibre vers la formation du sel de formiate. Ainsi, lorsque l'hydrogénation catalytique du $CO_2$ est choisie et que dans le procédé de l'invention la dismutation nécessite la présence d'une base, les deux réactions peuvent être réalisées à la suite dans le même réacteur.

Si l'électro-réduction à 2 $e^-$ du $CO_2$ est choisie pour préparer l'acide formique et la dismutation nécessite la présence d'une base, les deux réactions peuvent être réalisées à la suite dans le même réacteur. La base devra alors être ajoutée.

La réduction à 2$e^-$ du $CO_2$, est préférentiellement effectuée selon les méthodes les plus sélectives connues à ce jour. Par exemple, l'hydrogénation catalytique du $CO_2$ peut être effectuée selon le protocole de Nozaki *et coll.* [R. Tanaka, M. Yamashita, K. Nozaki, J. Am. Chem. Soc. 2009, 131, 14168-14169] et permet de récupérer du formiate de potassium. L'éléctro-réduction peut quant à elle être conduite selon les conditions de Shibata et coll. ou de Hori *et coll.* [Y. Hori, H. Wakebe, T. Tsukamoto, O. Koga, Electrochim. Acta 1994, 39, 1833-1839 ; N. Furuya, T. Yamazaki, M. Shibata, J. Electroanal. Chem. 1997, 431, 39-41]

Il est à noter que les électro-réductions sont dépendantes d'un grand nombre de paramètres tels que la nature du réacteur, les électrodes, l'électrolyte ou encore l'électro-catalyseur utilisé. Ces notions et les différents systèmes permettant d'obtenir de l'acide formique ou un de ses dérivés sont discutés dans les revues d'Olah et coll., de Leung, Xuan et *coll.* ou encore de Kenis et coll. [A. Goeppert, M. Czaun, J. P. Jones, G. K. Surya Prakash, G. A. Olah, Chem. Soc. Rev. 2014, 43, 7995-8048 ; H.-R. M. Jhong, S. Ma, P. J. A. Kenis, Curr. Opin. Chem. Eng. 2013, 2, 191-199 ; X. Lu, D. Y. C. Leung, H. Wang, M. K. H. Leung, J. Xuan, ChemElectroChem 2014, 1, 836-849]. L'acide formique ou l'un de ses dérivés (formiate de sodium, de potassium, de lithium, de cesium, d'ammonium, par exemple) ainsi formé peut ensuite être engagé dans l'étape de dismutation.

L'invention a pour objet un procédé de production de méthanol caractérisé en ce qu'il comprend

(A) une étape de préparation d'un méthoxyborane de formule (I) par dismutation de l'acide formique, ou d'au moins un de ses dérivés de formule $HCO_2M$ dans laquelle M est choisi dans le groupe formé par $Na^+$, $K^+$, $Li^+$, $Cs^+$, $NH_4^+$, le triéthylammonium ($HNEt_3^+$), le tétraphénylphosphonium ($PPh_4^+$), le tétraméthylammonium ($NMe_4^+$), le tétraéthylammonium ($NEt_4^+$), le tétrabutylammonium ($NBu_4^+$), et le tétraphénylammonium ($NPh_4^+$), ou un mélange d'acide formique et d'au moins un de ses dérivés,

en présence
d'un organoborane de formule (II) ; et éventuellement
d'une base organique, selon le procédé de l'invention ;
(B) une étape d'hydrolyse ou de protonolyse du méthoxyborane de formule (I) obtenu à l'issue de l'étape de dismutation (A) en méthanol.

Préalablement à l'étape (A), l'acide formique ou ses dérivés comme définis précédemment sont préparés, de préférence, par la réduction à $2e^-$ du $CO_2$, ou par l'hydrogénation catalytique du $CO_2$.

L'invention a également pour objet un procédé de production de méthanol caractérisé en ce qu'il comprend

(i) une étape de préparation de l'acide formique ou de ses dérivés de formule $HCO_2M$ dans laquelle M est choisi dans le groupe formé par $Na^+$, $K^+$, $Li^+$, $Cs^+$, $NH_4^+$, le triéthylammonium ($HNEt_3^+$), le tétraphénylphosphonium ($PPh_4^+$), le tétraméthylammonium ($NMe_4^+$), le tétraéthylammonium ($NEt_4^+$), le tétrabutylammonium ($NBu_4^+$), et le tétraphénylammonium ($NPh_4^+$), par la réduction à $2e^-$ du $CO_2$, ou par l'hydrogénation catalytique du $CO_2$ ;
(ii) une étape de préparation d'un méthoxyborane de formule (I) par dismutation de l'acide formique ou d'au moins un de ses dérivés, ou d'un mélange d'acide formique et au moins un de ses dérivés, selon le procédé de l'invention ; et
(iii) une étape d'hydrolyse ou de protonolyse du méthoxyborane de formule (I) obtenu à l'issue de l'étape de dismutation (ii) en méthanol.

Ce procédé est représenté en Figure 6. Les gaz générés dans ce procédé (essentiellement $CO_2$ et en moindre quantité $H_2$) peuvent être récupérés et réutilisés dans l'étape (i) du procédé. De la même manière, l'organoborane de formule (II) généré dans ce procédé peut être récupéré après l'étape de protonolyse et réutilisé dans l'étape de dismutation (ii).

Ce procédé de production de méthanol est un cas particulier du procédé de production de méthanol décrit précédemment. De ce fait, les étapes (ii) et (iii) de ce procédé de production de méthanol sont identiques aux étapes (A) et (B) du procédé de production de méthanol décrites plus haut. Ainsi, tout ce qui est décrit concernant notamment les conditions opératoires, les avantages et les particularités pour *les* étapes (ii) et (iii) de ce procédé, s'appliquent de la même manière aux étapes (A) et (B) du procédé décrit précédemment, et réciproquement. De la même manière, tout ce qui est décrit concernant notamment les conditions opératoires, les avantages et les particularités concernant la réaction de dismutation, s'applique au procédé de préparation d'un méthoxyborane de formule (I) et aux deux procédés de production de méthanol décrits.

L'étape (ii) ou (A) consiste à mettre en réaction :

- l'acide formique ou au moins un de ses dérivés comme précisés précédemment obtenus à l'étape (i) ou préalablement à l'étape (A), ou un mélange d'acide formique et au moins un de ses dérivés ;
- un organoborane de formule (II) ;
- éventuellement une base de préférence organique comme précisée précédemment.

Les différents réactifs utilisés dans les procédés de l'invention (les organoboranes de formule (II), l'acide formique ou ses dérivés, la base, l'additif, les acides etc.) sont, en général, des composés commerciaux ou peuvent être préparés par tout procédé connu de l'homme du métier.

La quantité de l'organoborane de formule (II) est de 0,05 à 1 équivalent molaire, de préférence de 0,5 à 1 équivalent molaire, bornes incluses, par rapport à l'acide formique ou à son(ses) dérivé(s) ou à un mélange d'acide formique et d'au moins un de ses dérivés.

Lorsqu'une base est utilisée, la quantité de base est de 0,05 à 3 équivalents molaires de préférence de 0,5 à 1,5 équivalents molaires bornes incluses, par rapport à l'acide formique, ou à son(ses) dérivé(s) ou à un mélange d'acide formique et d'au moins un de ses dérivés.

Lorsqu'un additif est utilisé, la quantité de l'additif est de 1 à 2 équivalents molaires de préférence de 1 à 1,5 équivalents molaires bornes incluses, par rapport au(x) dérivé(s) de l'acide formique ou à un mélange d'acide formique et d'au moins un de ses dérivés.

Le nombre d'équivalents de l'organoborane de formule (II), de la base et de l'additif sont en fait calculés par rapport au nombre d'équivalents de $HCOO^-$. Par exemple, dans le cas d'un mélange d'acide formique avec au moins un de ses dérivés comme par exemple, un mélange HCOOH/HCOONa (1:1), le nombre d'équivalents formels de $HCOO^-$ est de 2.

La réaction de dismutation génère une pression de gaz résultant de la formation de dioxyde de carbone et éventuellement de dihydrogène. La réaction peut alors se produire sous pression des gaz formés ou sous pression atmosphérique en collectant les gaz, par exemple dans une burette. Ce(s) gaz peuvent être réutilisés pour préparer l'acide formique ou l'un de ses dérivés par exemple dans l'étape (i) du procédé, à savoir pour effectuer la réduction du

$CO_2$ à $2e^-$.

La température de la réaction de dismutation peut être comprise entre 25 et 150°C, de préférence entre 25 et 130°C, bornes incluses.

La durée de la réaction dépend du taux de conversion de l'acide formique, La réaction peut être effectuée pendant une durée de 5 minutes à 200 heures, de préférence de 10 minutes à 48 heures, bornes incluses.

L'étape de dismutation (ii) ou (A) peut être effectuée dans l'acide formique pur, dans l'un des dérivés de l'acide formique si celui-ci est liquide ou en utilisant un ou un mélange d'au moins deux solvants. Le solvant peut être choisis dans le groupe formé par :

- l'eau ;
- les alcools, par exemple, l'éthanol ou l'éthylène glycol ;
- les éthers, par exemple, l'éther diéthylique, le THF, le diglyme, le 1,4-dioxane ;
- les hydrocarbures, par exemple, le benzène, ou le toluène ;
- les solvants azotés, par exemple, la pyridine, ou l'acétonitrile ;
- les sulfoxydes, par exemple, le diméthylesulfoxyde ;
- les halogénures d'alkyle, par exemple, le chloroforme, ou le chlorure de méthylène ; et
- un fluide supercritique, par exemple, le $CO_2$ supercritique.

De préférence, la réaction de dismutation est conduite dans un solvant polaire aprotique choisi parmi le THF ou l'acétonitrile. Plus préférentiellement, le solvant est l'acétonitrile.

L'étape d'hydrolyse ou la protonolyse (iii) ou (B) permet de transformer le méthoxyborane de formule (I) en méthanol libre $CH_3OH$, éventuellement en régénérant les organoboranes de formule (II).

Les organoboranes de formule (II) obtenus après l'étape de protonolyse ou d'hydrolyse des méthoxyboranes peuvent être réutilisés pour produire les méthoxyboranes par le procédé d'invention.

L'étape d'hydrolyse ou de protonolyse peut être réalisée avec tout acide connu de l'homme du métier, par exemple, l'acide formique, l'acide acétique ou l'acide chlorhydrique.

En fait, une fois les méthoxyboranes obtenus, deux possibilités sont envisageables pour former le méthanol libre : par simple réaction avec l'eau (hydrolyse) ou bien avec un acide plus fort (protonolyse) comme par exemple, l'acide formique, l'acide acétique ou l'acide chlorhydrique anhydre.

Le méthanol obtenu par les procédés de l'invention, peut convenir à être utilisé dans tout procédé utilisant le méthanol, notamment tout procédé visant à utiliser le méthanol comme carburant ou additif dans les moteurs à combustion, comme carburant dans les piles à combustibles à méthanol direct, comme moyen de stockage de l'hydrogène (E. Alberico, M. Nielsen, Chem. Comm. 2015, 51, 6714-6725), comme réactif pour la chimie fine ou encore comme réactif pour la chimie lourde.

Ainsi, l'invention a encore pour objet l'utilisation de méthoxyboranes de formule (I) obtenus par le procédé de l'invention,

- pour la production du méthanol,
- comme catalyseurs dans des réactions d'allylation,
- comme réactifs dans les réactions de couplage de Suzuki,
- comme réactif pour la chimie fine ou pour la chimie lourde.

Le méthanol produit par hydrolyse ou protonolyse des méthoxyboranes formés par le procédé d'invention, peut être utilisé comme carburant ou additif dans les moteurs à combustion, comme carburant dans les piles à combustibles à méthanol direct, comme moyen de stockage de l'hydrogène, comme réactif pour la chimie fine.

D'autres avantages et caractéristiques de la présente invention apparaîtront à la lecture des exemples ci-dessous donnés à titre illustratif et non limitatif et les figures annexées :

- la Figure 1 représente la réduction hexa-électronique (à 6 électrons *i.e.* 3 hydrures) du $CO_2$ en méthanol par hydrogénation catalytique (Équation (1)), et l'électro-réduction hexa-électronique du $CO_2$ en méthanol (Équation (2)) ;
- la Figure 2 représente la dismutation de l'acide fornique en $CO_2$ et méthanol ;
- la Figure 3 représente deux réactions de décomposition de l'acide formique qui sont en compétition avec la réaction de dismutation de l'acide formique en méthanol et $CO_2$. Ces deux réactions sont : la déshydrogénation de l'acide formique en $CO_2$ et $H_2$ (Équation (4)) et la déshydratation de l'acide formique en CO et $H_2O$ (Équation (5)) ;
- la Figure 4 représente des exemples de carbènes N-hétérocycliques ;
- la Figure 5 représente la préparation de l'acide formique une électro-réduction à 2 $e^-$ ou une hydrogénation catalytique du $CO_2$ ;

- la Figure 6 représente le procédé de production de méthanol comprenant (i) une étape de préparation de l'acide formique ou de l'un de ses dérivés par la réduction à 2e$^-$ du $CO_2$, ou par l'hydrogénation catalytique du $CO_2$; (ii) une étape de préparation d'un méthoxyborane de formule (I) par dismutation de l'acide formique ou d'au moins un de ses dérivés, ou d'un mélange d'acide formique et au moins un de ses dérivés ; et (iii) une étape d'hydrolyse ou de protonolyse du méthoxyborane de formule (I) obtenu à l'issue de l'étape de dismutation (ii) en méthanol ;
- la Figure 7 représente les réactions intervenant dans le calcul de rendements d'obtention des méthoxyboranes.

**EXEMPLES**

Un ensemble de résultats est présenté ci-dessous, donnant des exemples de dismutation de l'acide formique en méthoxyborane de formule (I) et $CO_2$ en présence d'un organoborane de formule (II). Les rendements sont obtenus par RMN $^1$H en intégrant les signaux du méthoxyborane de formule (I) ou du méthanol obtenu après hydrolyse ou protonolyse du méthoxyborane de formule (I) par rapport à ceux d'un étalon interne (mésitylène). Les rendements sont calculés en respectant la stœchiométrie de la réaction de dismutation à savoir qu'au mieux, 3 moles d'acide formique donnent au maximum 1 mol de méthanol (voire l'équation 3 de la Figure 2).

$$\rho(MeOBR^1R^2) = \frac{3 \times n(MeOBR^1R^2)}{n_0(HCOOZ)}$$

$n_0(MeOBR^1R^2)$ : quantité de matière en méthoxyborane déterminé par RMN $^1$H par rapport au mésitylène

$n_0(HCOOZ) = n_0(HCOOH) + n_0(HCOOM)$: quantité de matière totale en acide formique (HCOOH) et ses dérivés (HCOOM, M tel que défini plus haut) introduite initialement.

Dans notre cas, l'utilisation d'organoboranes impose les équations 6 et 7 (Figure 7). Les rendements sont néanmoins calculés en se basant sur l'équation 3.

Le rendement d'hydrolyse étant quantitatif, les rendements en méthoxyboranes de formule (I) et en méthanol sont égaux (Figure 6).

La réaction de dismutation de l'acide formique peut être effectuée selon le protocole expérimental suivant :

1. Sous atmosphère inerte, en boîte à gants, l'organoborane de formule (II), l'acide formique ou l'un de ses dérivés, ou un mélange d'acide formique et d'au moins un de ses dérivés et éventuellement, le solvant et/ou la base et/ou l'additif sont introduits dans un tube de Schlenk qui est ensuite scellé par un robinet J. Young. L'ordre d'introduction des réactifs n'a pas d'importance.
2. Le Schlenk est ensuite chauffé à une température comprise entre 25 et 150 °C (préférentiellement > 110°C) jusqu'à la conversion totale de l'acide formique (de 5 minutes à 72 heures de réaction). Le suivi de la réaction est assuré par RMN du proton $^1$H et/ou $^{13}$C et/ou $^{11}$B.
3. Lorsque la réaction est terminée (ce qui correspond à la disparition des signaux caractéristiques des protons du formiate H-COO$^-$ en RMN $^1$H), la pression dans le tube est libérée et le solvant ainsi que les composés volatils sont évaporés sous vide ($10^{-2}$ mbar).
Il est à noter que les gaz générés par la réaction (essentiellement $CO_2$ et en moindre quantité $H_2$) peuvent être récupérés et réutilisés pour générer l'acide formique par hydrogénation catalytique du $CO_2$.
4. Le résidu obtenu après évaporation des volatils est ensuite hydrolysé ou protonolysé respectivement avec $H_2O$ ou HCl anhydre (en solution dans l'éther diéthylique) pour libérer le méthanol libre qui peut être collecté aisément par distillation. Dans le cas ou HCl est employé, il est également possible de récupérer les chloroorganoboranes par distillation, ces derniers pouvant être réutilisés dans la réaction de dismutation du procédé.

**EXEMPLE 1: Préparation de MeOBBN à partir de BBNI, d'acide formique et de triéthylamine**

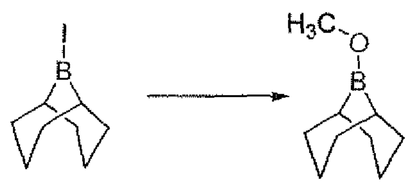

Selon le protocole général présenté ci-dessus, BBNI (commercial, 1 M dans l'hexane, 100 µL, 0,1 mmol) est ajouté à un mélange d'acide formique (7,4 µL, 0,2 mmol, 2 équiv.) et de triéthylamine $NEt_3$ (27,8 µL, 0,2 mmol, 2 équiv.) dans l'acétonitrile. Le tube est alors scellé et agité vigoureusement pour solubiliser le borane qui colle aux parois du tube. Une fois le mélange homogène (< 1 min), celui-ci est chauffé à 130°C pendant 19 h. Le mélange réactionnel est alors analysé par RMN $^1H$ et un rendement en MeOBBN de 19 % est obtenu. Ce dernier peut alors être hydrolysé pour obtenir le méthanol libre. Pour ce faire, le tube est ouvert et 18 µL d'eau (1 mmol, 5 équiv.) sont ajoutés. Le tube est alors agité pendant 30 min à température ambiante pour obtenir quantitativement le méthanol.

**EXEMPLE 2 : Préparation de $MeOBBu_2$ à partir de $Bu_2B$-OTf, d'acide formique et de triéthylamine**

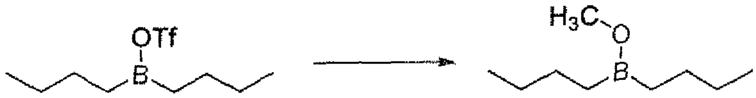

Par le même protocole que celui de l'exemple 1, en remplaçant BBNI par $Bu_2B$-OTf, un rendement de 15 % en méthanol boré $MeOBBu_2$ est obtenu.

**EXEMPLE 3 : Préparation de $MeOCy_2$ à partir de $Cy_2BCl$, d'acide formique et de triéthylamine**

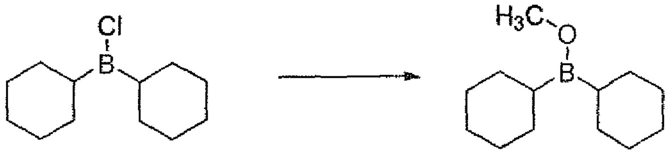

Par le même protocole que celui de l'exemple 1, en remplaçant BBNI par $Cy_2BCl$, un rendement de 17 % en méthanol boré $MeOBCy_2$ est obtenu.

**EXEMPLE 4: Préparation de $MeOBBu_2$ à partir de $Bu_3B$, d'acide formique et de triéthylamine**

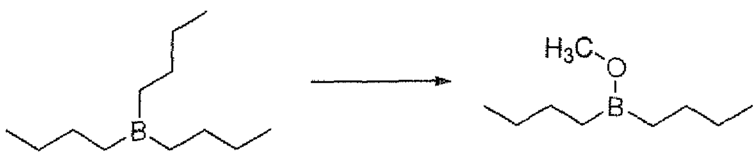

Par le même protocole que celui de l'exemple 1, en remplaçant BBNI par $Bu_3B$ (commercial, 1 M dans $Et_2O$) un rendement de **25 %** en méthoxyborane $MeOBBu_2$ est obtenu.

**EXEMPLE 5 : Préparation de MeOBBN à partir de BBNH, d'acide formique et de triéthylamine**

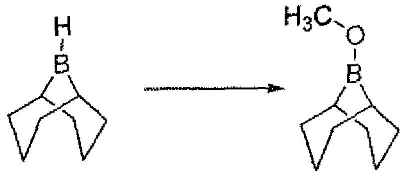

Selon le protocole général présenté ci-dessus, $(BBNH)_2$ (0,5 équiv.) est ajouté à un mélange d'acide formique

(2 équiv.) et de triéthylamine $NEt_3$ (1 équiv.) dans l'acétonitrile. Le tube est alors scellé et chauffé légèrement (*c.a. 50°C)* pour solubiliser les réactifs solides. Un fort dégagement d'hydrogène est alors observé, qui une fois terminé laisse un mélange homogène (*c.a.* 10 min). Celui-ci est chauffé à 130°C pendant 19 h. Le mélange réactionnel est alors analysé par RMN $^1H$ et un rendement en MeOBBN de 53 % est obtenu.

**EXEMPLE 6 : Préparation de MeOBBN à partir de BBNH, d'acide formique et de diisopropyléthylamine**

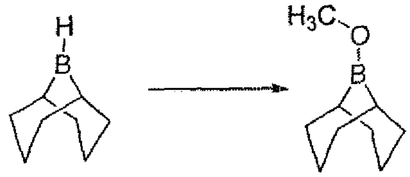

Selon le protocole général présenté ci-dessus, $(BBNH)_2$ (0,5 équiv.) est ajouté à un mélange d'acide formique (2 équiv.) et de diisopropyléthylamine *i*-$Pr_2NEt$ (1 équiv.) dans l'acétonitrile. Le tube est alors scellé et chauffé légèrement (*c.a. 50°C)* pour solubiliser les réactifs solides. Un fort dégagement d'hydrogène est alors observé, qui une fois terminé laisse un mélange homogène (*c.a.* 10 min). Celui-ci est chauffé à 130°C pendant 7h. Le mélange réactionnel est alors analysé par RMN $^1H$ et un rendement en MeOBBN de 49 % est obtenu.

**EXEMPLE 7 : Préparation de MeOBBN à partir de BBNH, d'acide formique et de formiate de sodium, en présence de l'éther couronne 15-C-5.**

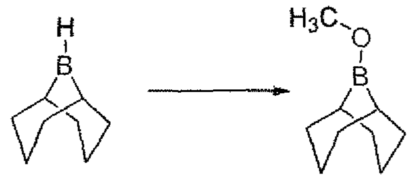

Selon le protocole général présenté ci-dessus, $(BBNH)_2$ (0,5 équiv.) est ajouté à un mélange d'acide formique (1 équiv.) et de formiate de sodium $HCO_2Na$ (1 équiv.) dans l'acétonitrile. L'éther couronne 15-C-5 (1 équiv.) est ensuite ajoutée à la seringue et le tube est scellé et chauffé légèrement (*c.a. 50°C*) pour solubiliser les réactifs solides. Celui-ci est chauffé à 130°C pendant 24 h. Le mélange réactionnel est alors analysé par RMN $^1H$ et un rendement en MeOBBN de 58 % est obtenu.

**EXEMPLE 8 : Préparation de $MeOBCy_2$ à partir de $Cy_2BH$, d'acide formique et de triéthylamine**

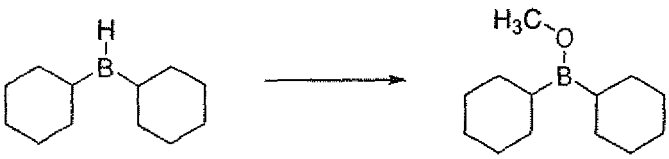

Selon le protocole général présenté ci-dessus, $Cy_2BH$ (1 équiv.) est ajouté à un mélange d'acide formique (2 équiv.) et de triéthylamine $NEt_3$ (1 équiv.) dans l'acétonitrile. Le tube est alors scellé et chauffé légèrement (*c.a. 50°C*) pour solubiliser les réactifs solides. Un fort dégagement d'hydrogène est alors observé, qui une fois terminé laisse un mélange homogène (*c.a.* 5 min). Celui-ci est chauffé à 120°C pendant 7h. Le mélange réactionnel est alors analysé par RMN $^1H$ et un rendement en $MeOBCy_2$ de 31 % est obtenu.

**EXEMPLE 9 : Production du méthanol**

Le MeOBBN obtenu dans l'exemple 5 peut alors être hydrolysé pour obtenir le méthanol libre :
Une fois la réaction terminée, les composés volatils sont évaporés sous vide ($10^{-1}$ à $10^{-2}$ mbar), le résidu solide obtenu est alors dissous dans du THF et $H_2O$ (5-10 équiv. par rapport au borane initialement introduit) est ajouté au mélange réactionnel. La solution est agitée 30 minutes à 1 heure à température ambiante (20 $\pm$ 5 °C). Le méthanol volatil est ensuite récupéré dans un autre tube de Schlenk par transfert sous pression réduite. On obtient ainsi une solution aqueuse de méthanol avec un rendement de 50 %.

## Revendications

1. Procédé de préparation de méthoxyboranes de formule (I)

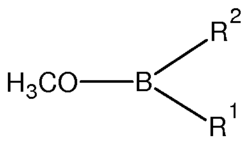

(I)

dans laquelle

- $R^1$ et $R^2$ indépendamment l'un de l'autre, sont choisis dans le groupe formé par un groupe hydroxyle, un groupe alcoxy, un groupe alkyle, un groupe alcényle, un groupe alcynyle, un groupe aryle, un groupe hétéroaryle, un groupe hétérocyclique, un atome d'halogène, un groupe silylé, un groupe siloxy, un groupe phosphino, et un groupe amino, lesdits groupes alkyle, alcényle, alcynyle, alcoxy, silyle, siloxy, aryle, hétéroaryle, hétérocyclique, phosphino et amino étant éventuellement substitués ;
- $R^1$ et $R^2$ pris ensemble avec l'atome de bore auquel ils sont liés, forment un hétérocycle éventuellement substitué ;

par dismutation de l'acide formique, ou d'au moins un de ses dérivés de formule $HCO_2M$ dans laquelle M est choisi dans le groupe formé par $Na^+$, $K^+$, $Li^+$, $Cs^+$, $NH_4^+$, le triéthylammonium ($HNEt_3^+$), le tétraphénylphosphonium ($PPh_4^+$), le tétraméthylammonium ($NMe_4^+$), le tétraéthylammonium ($NEt_4^+$), le tétrabutylammonium ($NBu_4^+$), et le tétraphénylammonium ($NPh_4^+$), ou d'un mélange d'acide formique et d'au moins un de ses dérivés de formule $HCO_2M$, en présence

- d'un organoborane de formule (II)

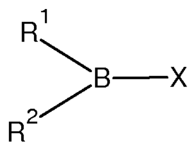

(II)

dans laquelle

- $R^1$ et $R^2$ sont tels que définis pour les composés méthoxyborane de formule (I) ;
- X est choisi dans le groupe formé par un atome d'hydrogène, un atome d'halogène, un groupe carboxylate, un groupe sulfonate, un groupe hydroxyle, un groupe alcoxy, un groupe alkyle, un groupe alcényle, un groupe alcynyle, un groupe aryle, un groupe hétéroaryle, un groupe hétérocyclique, un groupe silylé, un groupe siloxy, un groupe phosphino, un groupe amino, lesdits groupes alkyle, alcényle, alcynyle, alcoxy, silyle, siloxy, aryle, hétéroaryle, hétérocyclique, phosphino et amino étant éventuellement substitués ; et éventuellement

- d'une base organique choisie parmi :

- les bases organiques azotées choisies dans le groupe formé par la triéthylamine, la triméthylamine, la *N*-diisopropyléthylamine (DIPEA), la diéthylisopropylamine (DIEA), la 7-Méthyl-1,5,7-triazabicyclo[4.4.0]dec-5-ène (MTBD), le 1,8-diazabicyclo[5.4.0]undéc-7-ène (DBU), le 1,4-diazabicyclo[2.2.2]octane (DABCO), et la *N*-méthylpiperidine ;
- les bases organiques phosphorées choisies dans le groupe formé par la triphénylphosphine, le 2,2'-bis(diphénylphosphino)-1,1'-binaphthyle (BINAP), le triisopropylphosphine, 1,2-bis(diphénylphosphino)éthane (dppe), la tricyclohexylphosphine ($PCy_3$) ; les aza-phosphines choisies dans le groupe formé par le 2,8,9-triisopropyl-2,5,8,9-tétraaza-1-phosphabicyclo[3.3.3]undecane ($BV^{Me}$) et le 2,8,9-triisobutyl-2,5,8,9-tétraaza-1-phosphabicyclo[3.3.3]undecane ($BV^{iBu}$) ;
- les bases carbonées choisies parmi les carbènes N-hétérocycliques issus d'un sel d'imidazolium, ledits carbènes étant choisis dans le groupe formé par les sels de 1,3-bis(2,6-diisopropylphényl)-1H-imidazol-3-ium (également appelé IPr), 1,3-bis(2,6-diisopropylphényl)-4,5-dihydro-1H-imidazol-3-ium (également appelé s-IPr), 1,3-bis(2,4,6-triméthylphényl)-1H-imidazol-3-ium (également appelé IMes), 1,3-bis(2,4,6-triméthylphényl)-4,5-dihydro-1H-imidazol-3-ium (également s-IMes), 4,5-dichloro-1,3-bis(2,6-diisopropylphényl)-1H-imidazol-3-ium (également appelé $Cl_2$-IPr), 1,3-di-tert-butyl-1H-imidazol-3-ium (également appelé ItBu), et 1,3-di-tert-butyl-4,5-dihydro-1H-imidazol-3-ium (également appelé s-ItBu), lesdits sels étant sous la forme de sels de chlorure ou de tétraphénylborate ;

la quantité de l'organoborane de formule (II) est de 0,05 à 1 équivalent molaire, bornes incluses, par rapport à l'acide formique ou à son(ses) dérivé(s), ou à un mélange d'acide formique et d'au moins un de ses dérivés de formule $HCO_2M$, et la quantité de base est de 0,05 à 3 équivalents molaires, bornes incluses, par rapport à l'acide formique ou à son(ses) dérivé(s) de formule HCO2M, ou à un mélange d'acide formique et d'au moins un de ses dérivés de formule HCO2M;
un groupe alkyle désigne un radical carboné linéaire, ramifié ou cyclique, saturé, éventuellement substitué, comprenant 1 à 12 atomes de carbone;
par alcényle ou alcynyle, on entend un radical carboné insaturé linéaire, ramifié ou cyclique, éventuellement substitué, ledit radical carboné insaturé comprenant 2 à 12 atomes de carbone comprenant au moins une double (alcényle) ou une triple liaison (alcynyle)..

2. Procédé selon la revendication 1, **caractérisé en ce que** l'acide formique et ses dérivés, sont préparés par une électro-réduction à 2 $e^-$ ou une hydrogénation catalytique du $CO_2$.

3. Procédé selon l'une des revendications 1 ou 2, **caractérisé en ce que** dans le méthoxyborane de formule (I) et l'organoborane de formule (II), $R^1$ et $R^2$, indépendamment l'un de l'autre, sont choisis dans le groupe formé par un groupe alkyle comprenant 1 à 12 atomes de carbone ; un aryle comprenant 6 à 20 atomes de carbone, lesdits groupes alkyle et aryles étant éventuellement substitués.

4. Procédé selon l'une quelconque des revendications 1 à 3, **caractérisé en ce que** dans le méthoxyborane de formule (I) et l'organoborane de formule (II), $R^1$ et $R^2$ pris ensemble avec l'atome de bore auquel ils sont liés, forment un hétérocycle comportant 5 à 10 membres, ledit hétérocycle étant éventuellement substitué.

5. Procédé selon l'une quelconque des revendications 1 à 4, **caractérisé en ce que** dans l'organoborane de formule (II), X est choisi dans le groupe formé par un atome d'hydrogène ; un groupe carboxylate de formule $-OCOR^8$ dans laquelle $R^8$ est choisi parmi un atome d'hydrogène, un groupe alkyle comprenant 1 à 12 ; un atome d'halogène ; un groupe alkyle comprenant 1 à 12; un groupe sulfonate de formule $-OSO_2R^7$, dans lequel $R^7$ est choisi parmi un groupe méthyle ($CH_3$) un groupe trifluorométhyle ($CF_3$), un groupe toluène (*p*-$CH_3C_6H_4$) ou un groupe benzène ($C_6H_5$).

6. Procédé selon l'une quelconque des revendications 1 à 5, **caractérisé en ce que** l'organoborane de formule (II) est choisi dans le groupe formé par le tributylborane, le dicyclohexylborane, l'iododicyclohexylborane, le dibutylborane méthanesulfonate (n-$Bu_2BOSO_3Me$), le B-iodo-9-borabicyclo[3.3.1]nonane, le dimère de 9-borabicyclo[3.3.1]nonane, le B-benzyl-9-borabicyclo[3.3.1]nonane, le Me-TBD-$BBN^+I^-$.

7. Procédé selon l'une quelconque des revendications 1 à 6, **caractérisé en ce que** la base organique est une base organique azotée choisie dans le groupe formé par la triéthylamine, la triméthylamine, la *N*-diisopropyléthylamine (DIPEA), la diéthylisopropylamine (DIEA), la 7-Méthyl-1,5,7-triazabicyclo[4.4.0]dec-5-ène (MTBD), le 1,8-diazabicyclo[5.4.0]undéc-7-ène (DBU), le 1,4-diazabicyclo[2.2.2]octane (DABCO), et la *N*-méthylpiperidine.

8. Procédé selon l'une quelconque des revendications 1 à 7, **caractérisé en ce que** la dismutation de l'acide formique, ou d'au moins un de ses dérivés ou d'un mélange d'acide formique et d'au moins un de ses dérivés comme définis

à la revendication 1, a lieu, en outre, en présence d'un additif choisis parmi :

- les éthers couronnes dans le groupe formé par le 12-couronne-4, le 15-couronne-5, le 18-couronne-6, le dibenzo-18-couronne-6, le benzo-18-couronne-6, le benzo-15-couronne-5, et le dibenzo-15-couronne-5 ;
- les aza-couronnes dans le groupe formé par le 1,4,7,10-tétraazacyclododécane (cyclène), le 1,4,7,10,13,16-hexaazacyclooctadécane (hexacyclène), et le diaza-18-couronne-6 ;
- les thioéthers couronnes dans le groupe formé par le 1,5,9,13-tétrathiacyclohexadécane (16-Ane-$S_4$), et le 1,4,7,10,13,16-hexathiacyclooctadécane (18-Ane-$S_6$).

**9.** Procédé selon l'une quelconque des revendications 1 à 8, **caractérisé en ce que** la quantité de base est de 0,5 à 1,5 équivalents molaires, bornes incluses, par rapport à l'acide formique ou à son(ses) dérivé(s) de formule $HCO_2M$, ou à un mélange d'acide formique et d'au moins un de ses dérivés de formule $HCO_2M$.

**10.** Procédé selon l'une quelconque des revendications 1 à 9, **caractérisé en ce que** la quantité de l'additif est de 1 à 2 équivalents molaires, bornes incluses, par rapport au(x) dérivé(s) de formule $HCO_2M$ de l'acide formique, ou à un mélange d'acide formique et d'au moins un de ses dérivés de formule $HCO_2M$.

**11.** Procédé de production de méthanol **caractérisé en ce qu'**il comprend

(A) une étape de préparation d'un méthoxyborane de formule (I) par dismutation de l'acide formique, ou d'au moins un de ses dérivés de formule $HCO_2M$ dans laquelle M est choisi dans le groupe formé par $Na^+$, $K^+$, $Li^+$, $Cs^+$, $NH_4^+$, le triéthylammonium ($HNEt_3^+$), le tétraphénylphosphonium ($PPh_4^+$), le tétraméthylammonium ($NMe_4^+$), le tétraéthylammonium ($NEt_4^+$), le tétrabutylammonium ($NBu_4^+$), et le tétraphénylammonium ($NPh_4^+$), ou d'un mélange d'acide formique et d'au moins un de ses dérivés de formule $HCO_2M$,
en présence
d'un organoborane de formule (II) ; et éventuellement
d'une base organique,
selon l'une quelconque des revendications 1 à 10 ; et
(B) une étape d'hydrolyse ou de protonolyse du méthoxyborane de formule (I) obtenu à l'issue de l'étape de dismutation (A) en méthanol.

**12.** Procédé de production de méthanol **caractérisé en ce qu'**il comprend

(i) une étape de préparation de l'acide formique ou de ses dérivés de formule $HCO_2M$ dans laquelle M est choisi dans le groupe formé par $Na^+$, $K^+$, $Li^+$, $Cs^+$, $NH_4^+$, le triéthylammonium ($HNEt_3^+$), le tétraphénylphosphonium ($PPh_4^+$), le tétraméthylammonium ($NMe_4^+$), le tétraéthylammonium ($NEt_4^+$), le tétrabutylammonium ($NBu_4^+$), et le tétraphénylammonium ($NPh_4^+$), par la réduction à $2e^-$ du $CO_2$, ou par l'hydrogénation catalytique du $CO_2$ ;
(ii) une étape de préparation d'un méthoxyborane de formule (I) par dismutation de l'acide formique ou d'au moins un de ses dérivés de formule $HCO_2M$, ou d'un mélange d'acide formique et d'au moins un de ses dérivés de formule $HCO_2M$, en présence d'un organoborane de formule (II), et éventuellement d'une base organique, selon l'une quelconque des revendications 1 à 10 ; et
(iii) une étape d'hydrolyse ou de protonolyse du méthoxyborane de formule (I) obtenu à l'issue de l'étape de dismutation (ii) en méthanol.

## Patentansprüche

**1.** Herstellungsverfahren für Methoxyborane der Formel (I),

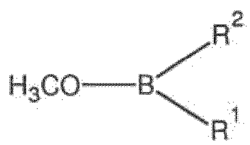

(I)

wobei

- $R^1$ und $R^2$ unabhängig voneinander ausgewählt sind aus der Gruppe, gebildet aus einer Hydroxylgruppe, einer Alkoxygruppe, einer Alkylgruppe, einer Alkenylgruppe, einer Alkinylgruppe, einer Arylgruppe, einer Heteroarylgruppe, einer heterocyclischen Gruppe, einem Halogenatom, einer silylierten Gruppe, einer Siloxygruppe, einer Phosphingruppe und einer Aminogruppe, wobei die Alkyl-, Alkenyl-, Alkinyl-, Alkoxy, Silyl-, Siloxy-, Aryl-, Heteroaryl-, heterocyclische, Phosphin- und Aminogruppen gegebenenfalls substituiert sind;
- $R^1$ und $R^2$ zusammengenommen mit dem Boratom, an das sie gebunden sind, einen gegebenenfalls substituierten Heterocyclus bilden;

durch Dismutation von Ameisensäure oder mindestens einem ihrer Derivate der Formel $HCO_2M$, wobei M ausgewählt ist aus der Gruppe, gebildet aus $Na^+$, $K^+$, $Li^+$, $Cs^+$, $NH_4^+$, Triethylammonium ($HNEt_3^+$), Tetraphenylphosphonium ($PPh_4^+$), Tetramethylammonium ($NMe_4^+$), Tetraethylammonium ($NEt_4^+$), Tetrabutylammonium ($NBu_4^+$) und Tetraphenylammonium ($NPh_4^+$), oder von einem Gemisch aus Ameisensäure und mindestens einem ihrer Derivate der Formel $HCO_2M$ in Gegenwart

- eines Organoborans der Formel (II)

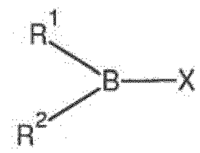

(II)

wobei

- $R^1$ und $R^2$ wie für die Methoxyboranverbindungen der Formel (I) definiert sind;
- X ausgewählt ist aus der Gruppe, gebildet aus einem Wasserstoffatom, einem Halogenatom, einer Carboxylatgruppe, einer Sulfonatgruppe, einer Hydroxylgruppe, einer Alkoxygruppe, einer Alkylgruppe, einer Alkenylgruppe, einer Alkinylgruppe, einer Arylgruppe, einer Heteroarylgruppe, einer heterocyclischen Gruppe, einer silylierten Gruppe, einer Siloxygruppe, einer Phosphingruppe, einer Aminogruppe, wobei die Alkyl-, Alkenyl-, Alkinyl-, Alkoxy-, Silyl-, Siloxy-, Aryl-, Heteroaryl-, heterocyclische, Phosphin- und Aminogruppe gegebenenfalls substituiert sind; und gegebenenfalls

- einer organischen Base, ausgewählt aus:

- organischen stickstoffhaltigen Basen, ausgewählt aus der Gruppe, gebildet aus Triethylamin, Trimethylamin, *N*-Diisopropylethylamin (DIPEA), Diethylisopropylamin (DIEA), 7-Methyl-1,5,7-triazabicyclo[4.4.0]dec-5-en (MTBD), 1,8-Diazabicyclo[5.4.0]undec-7-en (DBU), 1,4-Diazabicyclo[2.2.2]octan (DABCO) und *N*-Methylpiperidin;
- organischen phosphorhaltigen Basen, ausgewählt aus der Gruppe, gebildet aus Triphenylphosphin, 2,2'-Bis(diphenylphosphino)-1,1'-binaphthyl (BINAP), Triisopropylphosphin, 1,2-Bis(diphenylphosphino)ethan (dppe), Tricyclohexylphosphin ($PCy_3$); Azaphosphinen, ausgewählt aus der Gruppe, gebildet aus 2,8,9-Triisopropyl-2,5,8,9-tetraaza-1-phosphabicyclo[3.3.3]undecan ($BV^{Me}$[A1]) und 2,8,9-Triisobutyl-2,5,8,9-tetraaza-1-phosphabicyclo[3.3.3]undecan ($BV^{iBu}$);
- kohlenstoffhaltigen Basen, ausgewählt aus N-heterocyclischen Carbenen, die aus einem Imidazoliumsalz stammen, wobei die Carbene ausgewählt sind aus der Gruppe, gebildet aus den Salzen von 1,3-Bis(2,6-diisopropylphenyl)-1H-imidazol-3-ium (auch IPr genannt), 1,3-Bis(2,6-diisopropylphenyl)-4,5-dihydro-1H-imidazol-3-ium (auch s-IPr genannt), 1,3-Bis(2,4,6-trimethylphenyl)-1H-imidazol-3-ium (auch IMes genannt), 1,3-Bis(2,4,6-trimethylphenyl)-4,5-dihydro-1H-imidazol-3-ium (auch s-IMes genannt), 4,5-Dichlor-1,3-bis(2,6-diisopropylphenyl)-1H-imidazol-3-ium (auch $Cl_2$-IPr genannt), 1,3-Di-tert-butyl-1H-imidazol-3-ium (auch ItBu genannt) und 1,3-Di-tert-butyl-4,5-dihydro-1H-imidazol-3-ium (auch s-ItBu genannt), wobei die Salze in Form von Chlor- oder Tetraphenylboratsalzen vorliegen;

wobei die Menge des Organoborans der Formel (II) von einschließlich 0,05 bis einschließlich 1 Moläquivalent, bezogen auf die Ameisensäure oder ihr(e) Derivat(e) oder auf ein Gemisch von Ameisensäure und mindestens einem ihrer Derivate der Formel $HCO_2M$, beträgt, und
die Menge der Base von einschließlich 0,05 bis einschließlich 3 Moläquivalente, bezogen auf die Ameisensäure oder auf ihr(e) Derivat(e) der Formel HCO2M oder auf ein Gemisch von Ameisensäure und mindestens einem ihrer

Derivate der Formel HCO2M, beträgt.

2. Verfahren nach Anspruch 1, **dadurch gekennzeichnet, dass** die Ameisensäure und ihre Derivate durch 2e$^-$-Elektroreduktion oder eine katalytische Hydrierung des $CO_2$ hergestellt werden.

3. Verfahren nach einem der Ansprüche 1 oder 2, **dadurch gekennzeichnet, dass** in dem Methoxyboran der Formel (I) und dem Organoboran der Formel (II) $R^1$ und $R^2$ unabhängig voneinander ausgewählt sind aus der Gruppe, gebildet aus einer Alkylgruppe, die 1 bis 12 Kohlenstoffatome umfasst; einem Aryl, das 6 bis 20 Kohlenstoffatome umfasst, wobei die Alkyl- und Arylgruppe gegebenenfalls substituiert sind.

4. Verfahren nach einem der Ansprüche 1 bis 3, **dadurch gekennzeichnet, dass** in dem Methoxyboran der Formel (I) und dem Organoboran der Formel (II) $R^1$ und $R^2$ zusammengenommen mit dem Boratom, an das sie gebunden sind, einen Heterocyclus, enthaltend 5 bis 10 Glieder, bilden, wobei der Heterocyclus gegebenenfalls substituiert ist.

5. Verfahren nach einem der Ansprüche 1 bis 4, **dadurch gekennzeichnet, dass** in dem Organoboran der Formel (II) X ausgewählt ist aus der Gruppe, gebildet aus einem Wasserstoffatom; einer Carboxylatgruppe der Formel -$OCOR^8$, wobei $R^8$ ausgewählt ist aus einem Wasserstoffatom, einer Alkylgruppe umfassend 1 bis 12; einem Halogenatom; einer Alkylgruppe umfassend 1 bis 12; einer Sulfonatgruppe der Formel -$OSO_2R^7$, wobei $R^7$ aus einer Methylgruppe ($CH_3$), einer Trifluormethylgruppe ($CF_3$), einer Toluolgruppe ($p$-$CH_3C_6H_4$) oder einer Benzolgruppe ($C_6H_5$) ausgewählt ist.

6. Verfahren nach einem der Ansprüche 1 bis 5, **dadurch gekennzeichnet, dass** das Organoboran der Formel (II) ausgewählt ist aus der Gruppe, gebildet aus Tributylboran, Dicyclohexylboran, Ioddicyclohexylboran, Dibutylboranmethansulfonat ($n$-$Bu_2BOSO_3Me$), B-Iod-9-borabicyclo[3.3.1]nonan, dem Dimer von 9-Borabicyclo[3.3.1]nonan, B-Benzyl-9-borabicyclo[3.3.1]nonan, Me-TBD-$BBN^+I^-$.

7. Verfahren nach einem der Ansprüche 1 bis 6, **dadurch gekennzeichnet, dass** die organische Base eine organische stickstoffhaltige Base ist, ausgewählt aus der Gruppe, gebildet aus Triethylamin, Trimethylamin, *N*-Diisopropylethylamin (DIPEA), Diethylisopropylamin (DIEA), 7-Methyl-1,5,7-triazabicyclo[4.4.0]dec-5-en (MTBD), 1,8-Diazabicyclo[5.4.0]undec-7-en (DBU), 1,4-Diazabicyclo[2.2.2]octan (DABCO) und *N*-Methylpiperidin.

8. Verfahren nach einem der Ansprüche 1 bis 7, **dadurch gekennzeichnet, dass** die Dismutation der Ameisensäure oder mindestens einem ihrer Derivate oder eines Gemischs von Ameisensäure und mindestens einem ihrer Derivate wie in Anspruch 1 definiert weiter in Gegenwart eines Zusatzstoffs stattfindet, der ausgewählt ist aus:

- den Kronenethern aus der Gruppe, gebildet aus 12-Krone-4, 15-Krone-5, 18-Krone-6, Dibenzo-18-krone-6, Benzo-18-krone-6, Benzo-15-krone-5 und Dibenzo-15-krone-5;
- den Azakronen aus der Gruppe, gebildet aus 1,4,7,10-Tetraazacyclododecan (Cyclen), 1,4,7,10,13,16-Hexaazacyclooctadecan (Hexacyclen) und Diaza-18-krone-6;
- den Thiokronenethern aus der Gruppe, gebildet aus 1,5,9,13-Tetrathiacyclohexadecan (16-Ane-$S_4$) und 1,4,7,10,13,16-Hexathiacyclooctadecan (18-Ane-$S_6$).

9. Verfahren nach einem der Ansprüche 1 bis 8, **dadurch gekennzeichnet, dass** die Menge der Base von einschließlich 0,5 bis einschließlich 1,5 Moläquivalente, bezogen auf die Ameisensäure oder ihr(e) Derivat(e) der Formel $HCO_2M$ oder auf ein Gemisch von Ameisensäure und mindestens einem ihrer Derivate der Formel $HCO_2M$, beträgt.

10. Verfahren nach einem der Ansprüche 1 bis 9, **dadurch gekennzeichnet, dass** die Menge des Zusatzstoffs von einschließlich 1 bis einschließlich 2 Moläquivalente, bezogen auf das (die) Derivat(e) der Formel $HCO_2M$ der Ameisensäure oder auf ein Gemisch von Ameisensäure und mindestens einem ihrer Derivate der Formel $HCO_2M$, beträgt.

11. Herstellungsverfahren für Methanol, **dadurch gekennzeichnet, dass** es umfasst

(A) einen Zubereitungsschritt eines Methoxyborans der Formel (I) durch Dismutation der Ameisensäure oder von mindestens einem ihrer Derivate der Formel $HCO_2M$, wobei M ausgewählt ist aus der Gruppe, gebildet aus $Na^+$, $K^+$, $Li^+$, $Cs^+$, $NH_4^+$, Triethylammonium ($HNEt_3^+$), Tetraphenylphosphonium ($PPh_4^+$), Tetramethylammonium ($NMe_4^+$), Tetraethylammonium ($NEt_4^+$), Tetrabutylammonium ($NBu_4^+$) und Tetraphenylammonium ($NPh_4^+$) oder einem Gemisch von Ameisensäure und mindestens einem ihrer Derivate der Formel $HCO_2M$, in Gegenwart

eines Organoborans der Formel (II); und gegebenenfalls einer organischen Base nach einem der Ansprüche 1 bis 10; und
(B) einen Hydrolyse- oder Protonolyseschritt des Methoxyborans der Formel (I), erhalten am Ende des Dismutationsschritts (A), zu Methanol.

**12.** Herstellungsverfahren für Methanol, **dadurch gekennzeichnet, dass** es umfasst

(i) einen Zubereitungsschritt der Ameisensäure oder ihrer Derivate der Formel $HCO_2M$, wobei M ausgewählt ist aus der Gruppe, gebildet aus $Na^+$, $K^+$, $Li^+$, $Cs^+$, $NH_4^+$, Triethylammonium ($HNEt_3^+$), Tetraphenylphosphonium ($PPh_4^+$), Tetramethylammonium ($NMe_4^+$), Tetraethylammonium ($NEt_4^+$), Tetrabutylammonium ($NBu_4^+$) und Tetraphenylammonium ($NPh_4^+$), durch $2e^-$-Reduktion des $CO_2$ oder durch katalytische Hydrierung des $CO_2$;
(ii) einen Zubereitungsschritt eines Methoxyborans der Formel (I) durch Dismutation der Ameisensäure oder mindestens eines ihrer Derivate der Formel $HCO_2M$ oder eines Gemischs von Ameisensäure und mindestens einem ihrer Derivate der Formel $HCO_2M$ in Gegenwart eines Organoborans der Formel (II) und gegebenenfalls einer organischen Base nach einem der Ansprüche 1 bis 10; und
(iii) einen Hydrolyse- oder Protonolyseschritt des Methoxyborans der Formel (I), erhalten am Ende des Dismutationsschritts (ii), zu Methanol.

## Claims

**1.** Method for preparing methoxyboranes of formula (I)

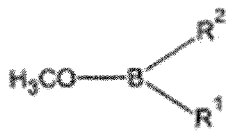

(I)

wherein

- $R^1$ and $R^2$, independently of one another, are selected from the group formed by a hydroxyl group, an alkoxy group, an alkyl group, an alkenyl group, an alkynyl group, an aryl group, a heteroaryl group, a heterocyclic group, a halogen atom, a silyl group, a siloxy group, a phosphine group, and an amino group, said alkyl, alkenyl, alkynyl, alkoxy, silyl, siloxy, aryl, heteroaryl, heterocyclic, phosphine and amino groups being optionally substituted;
- $R^1$ and $R^2$ taken together with the boron atom to which they are bonded, form an optionally substituted heterocycle;

by dismutation of formic acid, or of at least one of the derivatives thereof of formula $HCO_2M$ wherein M is selected from the group formed by $Na^+$, $K^+$, $Li^+$, $Cs^+$, $NH_4^+$, triethylammonium ($HNEt_3^+$), tetraphenylphosphonium ($PPh_4^+$), tetramethylammonium ($NMe_4^+$), tetraethylammonium ($NEt_4^+$), tetrabutylammonium ($NBu_4^+$), and tetraphenylammonium ($NPh_4^+$), or a mixture of formic acid and at least one of the derivatives thereof of formula $HCO_2M$, in presence

- of an organoborane of formula (II)

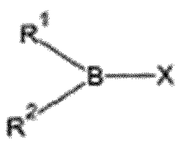

(II)

wherein

- $R^1$ and $R^2$ are as defined for the methoxyborane of formula (I);

- X is selected from the group formed by a hydrogen atom, a halogen atom, a carboxylate group, a sulfonate group, a hydroxyl group, an alkoxy group, an alkyl group, an alkenyl group, an alkynyl group, an aryl group, a heteroaryl group, a heterocyclic group, a silyl group, a siloxy group, a phosphine group, an amino group, said alkyl, alkenyl, alkynyl, alkoxy, silyl, siloxy, aryl, heteroaryl, heterocyclic, phosphine and amino groups being optionally substituted; and optionally

▪ of an organic base selected from:

- nitrogenous organic bases from the group formed by triethylamine, trimethylamine, *N*-diisopropylethylamine (DIPEA), diethylisopropylamine (DIEA), 7-Methyl-1,5,7-triazabicyclo[4.4.0]dec-5-ene (MTBD), 1,8-diazabicyclo[5.4.0]undec-7-ene (DBU), 1,4-diazabicyclo[2.2.2]octane (DABCO), and *N*-methylpiperidine;
- organic phosphorus bases selected from the group formed by triphenylphosphine, 2,2'-bis(diphenylphosphino)-1,1'-binaphthyl (BINAP), triisopropylphosphine, 1,2-bis(diphenylphosphino)ethane (dppe), tricylcohexylphosphine ($PCy_3$), aza-phosphines selected from the group formed by 2,8,9-triisopropyl-2,5,8,9-tetraaza-1-phosphabicyclo[3.3.3]undecane ($BV^{Me}$) and 2,8,9-triisobutyl-2,5,8,9-tetraaza-1-phosphabicyclo[3.3.3]undecane ($BV^{iBu}$);
- carbon bases selected from N-heterocyclic carbenes derived from an imidazolium salt, said carbenes being selected from the group formed by salts of 1,3-bis(2,6-diisopropylphenyl)-1H-imidazol-3-ium (also called IPr), 1,3-bis(2,6-diisopropylphenyl)-4,5-dihydro-1H-imidazol-3-ium (also called s-IPr), 1,3-bis(2,4,6-trimethylphenyl)-1H-imidazol-3-ium (also called IMes), 1,3-bis(2,4,6-trimethylphenyl)-4,5-dihydro-1H-imidazol-3-ium (also called s-IMes), 4,5-dichloro-1,3-bis(2,6-diisopropylphenyl)-1H-imidazol-3-ium (also called $Cl_2$-IPr), 1,3-di-tert-butyl-1H-imidazol-3-ium (also called ItBu), and 1,3-di-tert-butyl-4,5-dihydro-1H-imidazol-3-ium (also called s-ItBu), said salts being in the form of chloride or tetraphenylborate salts;

the quantity of organoborane of formula (II) is 0.05 to 1 molar equivalent, limits included, with respect to the formic acid or to the derivate(s) thereof, or a mixture of formic acid and of at least one of the derivatives thereof of formula $HCO_2M$, and the base quantity and 0.05 to 3 molar equivalents, limits included, with respect to the formic acid or to the derivative(s) thereof of formula $HCO_2M$, or to a mixture of formic acid and of at least one of the derivatives thereof of formula $HCO_2M$.

2. Method according to claim 1, **characterised in that** the formic acid and the derivatives thereof, are prepared by a $2e^-$ electro-reduction or a catalytic hydrogenation of $CO_2$.

3. Method according to one of claims 1 or 2, **characterised in that** in the methoxyborane of formula (I) and the organoborane of formula (II), $R^1$ and $R^2$, independently from one another, are selected from the group formed by an alkyl group comprising 1 to 12 carbon atoms; an aryl comprising 6 to 20 carbon atoms, said alkyl and aryl groups being possibly substituted.

4. Method according to any one of claims 1 to 3, **characterised in that** in the methoxyborane of formula (I) and the organoborane of formula (II), $R^1$ and $R^2$ taken together with the boron atom to which they are bound, form a heterocycle containing 5 to 10 members, said heterocycle being optionally substituted.

5. Method according to any one of claims 1 to 4, **characterised in that** in the organoborane of formula (II), X is selected from the group formed by a hydrogen atom; a carboxylate group of formula $-OCOR^8$ wherein $R^8$ is selected from a hydrogen atom, an alkyl group comprising 1 to 12; a halogen atom; an alkyl group comprising 1 to 12; a sulfonate group for formula $-OSO_2R^7$, wherein $R^7$ is selected from among a methyl group ($CH_3$), a trifluoromethyl group ($CF_3$), a toluene group ($p$-$CH_3C_6H_4$) or a benzene group ($C_6H_5$).

6. Method according to any one of claims 1 to 5, **characterised in that** the organoborane of formula (II) is selected from the group formed by tributylborane, dicyclohexylborane, iododicyclohexylborane, dibutyloborane methanesulfonate ($n$-$Bu_2BOSO_3Me$), B-iodo-9-borabicyclo[3.3.1]nonane, 9-borabicyclo[3.3.1]nonane dimer, B-benzyl-9-borabicyclo[3.3.1]nonane, Me-TBD-$BBN^+I^-$.

7. Method according to any one of claims 1 to 6, **characterised in that** the organic base is a nitrogenous organic base selected from the group formed by triethylamine, trimethylamine, *N*-diisopropylethylamine (DIPEA), diethylisopropylamine (DIEA), 7-Methyl-1,5,7-triazabicyclo[4.4.0]dec-5-ene (MTBD), 1,8-diazabicyclo[5.4.0]undec-7-ene (DBU), 1,4-diazabicyclo[2.2.2]octane (DABCO), and *N*-methylpiperidine.

**8.** Method according to any one of claims 1 to 7, **characterised in that** the dismutation of the formic acid, or of at least one of the derivatives thereof or a mixture of formic acid and of at least one of the derivatives thereof as defined in claim 1, further occurs in the presence of an additive selected from:

- crown ethers from the group formed by 12-crown-4, 15-crown-5, 18-crown-6, dibenzo-18-crown-6, benzo-18-crown-6, benzo-15-crown-5, dibenzo-15-crown-5;
- aza-crowns from the group formed by 1,4,7,10-tetraazacyclododecane (cyclene), 1,4,7,10,13,16-hexaazacyclooctadecane (hexacyclene), and diaza-18-crown-6;
- crown thioethers from the group formed by 1,5,9,13-tetrathiacyclohexadecane (16-Ane-$S_4$), and 1,4,7,10,13,16-hexathiacyclooctadecane (18-Ane-$S_6$).

**9.** Method according to any one of claims 1 to 8, **characterised in that** the base quantity is 0.5 to 1.5 molar equivalents, limits included, with respect to the formic acid or to the derivative(s) thereof of formula $HCO_2M$, or to a mixture of formic acid and of at least one of the derivatives thereof of formula $HCO_2M$.

**10.** Method according to any one of claims 1 to 9, **characterised in that** the quantity of additive is 1 to 2 molar equivalents, limits included, with respect to the derivative(s) thereof of formula $HCO_2M$ of the formic acid, or to a mixture of formic acid and of at least one of the derivatives thereof of formula $HCO_2M$.

**11.** Method for producing methanol **characterised in that** it comprises

(A) a step of preparing a methoxyborane of formula (I) by dismutation of the formic acid, or of at least one of the derivatives thereof of formula $HCO_2M$ wherein M is selected from the group formed by $Na^+$, $K^+$, $Li^+$, $Cs^+$, $NH_4^+$, triethylammonium ($HNEt_3^+$), tetraphenylphosphonium ($PPh_4^+$), tetramethylammonium ($NMe_4^+$), tetraethylammonium ($NEt_4^+$), tetrabutylammonium ($NBu_4^+$), and tetraphenylammonium ($NPh_4^+$), or a mixture of formic acid and at least one of the derivatives thereof of formula $HCO_2M$,
in the presence
of an organoborane of formula (II); and optionally
of an organic base, according to any one of claims 1 to 10; and
(B) a step of hydrolysis or protonolysis of the methoxyborane of formula (I) obtained from the methanol dismutation step (A).

**12.** Method for producing methanol, **characterised in that** it comprises

(i) a step of preparing the formic acid or the derivatives thereof of formula $HCO_2M$, wherein M is selected from the group formed by $Na^+$, $K^+$, $Li^+$, $Cs^+$, $NH_4^+$, triethylammonium ($HNEt_3^+$), tetraphenylphosphonium ($PPh_4^+$), tetramethylammonium ($NMe_4^+$), tetraethylammonium ($NEt_4^+$), tetrabutylammonium ($NBu_4^+$), and tetraphenylammonium ($NPh_4^+$), by a $2e^-$ reduction of $CO_2$ or by the catalytic hydrogenation of $CO_2$;
(ii) a step of preparing a methoxyborane of formula (I) by dismutation of the formic acid or of at least one of the derivatives thereof of formula $HCO_2M$, or of a mixture of formic acid and of at least one of the derivatives thereof of formula $HCO_2M$, in the presence of an organoborane of formula (II), and optionally of an organic base, according to any one of claims 1 to 10; and
(iii) a step of hydrolysis or protonolysis of the methoxyborane of formula (I) obtained from the methanol dismutation step (ii).

$$CO_{2(g)} + 3\ H_{2(g)} \xrightarrow{\text{catalyseur}} CH_3OH_{(l)} + H_2O_{(l)} \quad \text{(Equation 1)}$$

$$CO_{2(g)} + 6\ H^+ + 6\ e^- \xrightarrow{\text{catalyseur}} CH_3OH_{(l)} + H_2O_{(l} \quad \text{(Equation 2)}$$

**Figure 1**

$$3\ HCO_2H_{(l)} \xrightarrow{\text{catalyseur}} CH_3OH_{(l)} + 2\ CO_{2(g)} + H_2O_{(l)} \quad (3)$$

(Equation 3)

**Figure 2**

$$HCO_2H_{(l)} \xrightarrow{\text{catalyseur}} CO_{2(g)} + H_{2(g)} \quad \text{(Equation 4)}$$

$$HCO_2H_{(l)} \xrightarrow{\text{catalyseur}} CO_{(g)} + H_2O_{(l)} \quad \text{(Equation 5)}$$

**Figure 3**

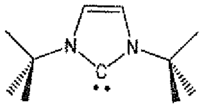
ItBu

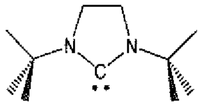
s-ItBu

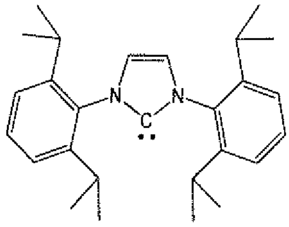
IPr

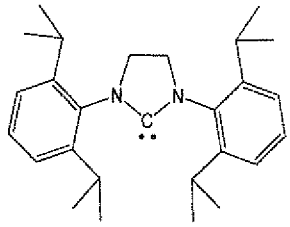
s-IPr

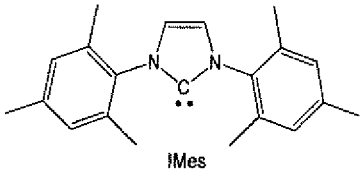
IMes

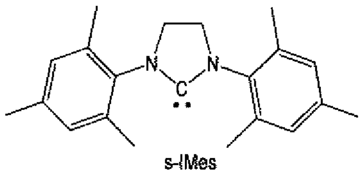
s-IMes

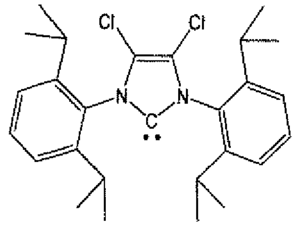
$Cl_2$-IPr

**Figure 4**

$$\begin{array}{c} CO_2 + 2\,e^- + 2H^+ \\ \text{ou} \\ CO_2 + H_2 \end{array} \longrightarrow \begin{array}{c} HCO_2H \\ \text{ou} \\ HCO_2^-,\ \text{Base } H^+ \end{array}$$

**Figure 5**

$$\begin{array}{c} CO_2 + 2\,e^- + 2H^+ \\ \text{ou} \\ CO_2 + H_2 \end{array} \xrightarrow{(i)} \begin{array}{c} HCO_2H \\ \text{ou} \\ HCO_2^-,\ \text{Base } H^+ \end{array} \xrightarrow[\begin{array}{c} BXR^1R^2 \\ (II) \end{array}]{(ii)} \begin{array}{c} 1/3\ CH_3OBR^1R^2 \\ (I) \\ + \\ 2/3\ CO_2 \end{array} \xrightarrow[\begin{array}{c} \text{hydrolyse ou} \\ \text{protonolyse} \end{array}]{(iii)} \begin{array}{c} 1/3\ CH_3OH \\ + \\ 1/3\ BXR^1R^2 \\ (II) \end{array}$$

**Figure 6**

$$3\ HCO_2H + 3\ R_1R_2X_1B \longrightarrow H_3COBR_1R_2 + R_1R_2BOBR_1R_2 + 2\ CO_2 + 3\ HX_1$$

(Equation 6)

$$6\ HCO_2H + 3\ R_1R_2X_1B \longrightarrow H_3COBR_1R_2 + R_1R_2BOBR_1R_2 + 5\ CO_2 + 3\ H_2 + 3\ HX_1$$

(Equation 7)

**Figure 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **K.-I. TOMINAGA ; Y. SASAKI ; M. KAWAI ; T. WATANABE ; M. SAITO.** *J. Chem. Soc., Chem. Commun.,* 1993, 629
- **C. A. HUFF ; M. S. SANFORD.** *J. Am. Chem. Soc.,* 2011, vol. 133, 18122-18125
- **S. WESSELBAUM ; T. VOM STEIN ; J. KLANKERMAYER ; W. LEITNER.** *Angew. Chem. Int Ed. Engl.,* 2012, vol. 51, 7499-7502
- **E. ALBERICO ; M. NIELSEN.** *Chem. Comm.,* 2015, vol. 51, 6714-6725
- **Y.-N. LI ; R. MA ; L.-N. HE ; Z.-F. DIAO.** *Catal. Sci. Technol.,* 2014, vol. 4, 1498-1512
- **A. GOEPPERT ; M. CZAUN ; J. P. JONES ; G. K. SURYA PRAKASH ; G. A. OLAH.** *Chem. Soc. Rev.,* 2014, vol. 43, 7995-8048
- **D. W. STEPHAN.** *Org. Biomol. Chem.,* 2008, vol. 6, 1535-1539
- **G. C. WELCH ; R. R. SAN JUAN ; J. D. MASUDA ; D. W. STEPHAN.** *Science,* 2006, vol. 314, 1124-1126
- **A. E. ASHLEY ; A. L. THOMPSON ; D. O'HARE.** *Angew. Chem. Int. Ed. Engl.,* 2009, vol. 48, 9839-9843
- **M. A. COURTEMANCHE ; A. P. PULIS ; E. ROCHETTE ; M. A. LEGARE ; D. W. STEPHAN ; F. G. FONTAINE.** *Chem. Comm.,* 2015, vol. 51, 9797-9800
- **G. SESHADRI ; C. LIN ; A. B. BOCARSLY.** *J. Electroanal. Chem.,* 1994, vol. 372, 145-150
- **H.-R. M. JHONG ; S. MA ; P. J. A. KENIS.** *Curr. Opin. Chem. Eng.,* 2013, vol. 2, 191-199
- **R. TANAKA ; M. YAMASHITA ; K. NOZAKI.** *J. Am. Chem. Soc.,* 2009, vol. 131, 14168-14169
- **C. FEDERSEL ; R. JACKSTELL ; M. BELLER.** *Angew. Chem. Int. Ed. Engl.,* 2010, vol. 49, 6254-6257
- **C. ZIEBART ; C. FEDERSEL ; P. ANBARASAN ; R. JACKSTELL ; W. BAUMANN ; A. SPANNENBERG ; M. BELLER.** *J. Am. Chem. Soc.,* 2012, vol. 134, 20701-20704
- **S. MORET ; P. J. DYSON ; G. LAURENCZY.** *Nat. Commun.,* 2014, vol. 5, 4017
- **A. S. AGARWAL ; Y. ZHAI ; D. HILL ; N. SRIDHAR.** *ChemSusChem,* 2011, vol. 4, 1301-1310
- **X. LU ; D. Y. C. LEUNG ; H. WANG ; M. K. H. LEUNG ; J. XUAN.** *ChemElectroChem,* 2014, vol. 1, 836-849
- **B. I. PODLOVCHENKO ; E. A. KOLYADKO ; S. LU.** *J. Electroanal. Chem.,* 1994, vol. 373, 185-187
- **A. J. MILLER ; D. M. HEINEKEY ; J. M. MAYER ; K. I. GOLDBERG.** *Angew. Chem. Int. Ed. Engl.,* 2013, vol. 52, 3981-3984
- **S. SAVOUREY ; G. LEFEVRE ; J. C. BERTHET ; P. THUERY ; C. GENRE ; T. CANTAT.** *Angew. Chem. Int. Ed. Engl.,* 2014, vol. 53, 10466-10470
- **M. C. NEARY ; G. PARKIN.** *Chem. Sci.,* 2015, vol. 6, 1859-1865
- **MARC-ANDRE COURTEMANCHE et al.** Reducing CO 2 to Methanol Using Frustrated Lewis Pairs: On the Mechanism of Phosphine-Borane-Mediated Hydroboration of CO 2. *JOURNAL OF THE AMERICAN CHEMICAL SOCIETY,* 2014, vol. 136 (30
- **Y. HORI ; H. WAKEBE ; T. TSUKAMOTO ; O. KOGA.** *Electrochim. Acta,* 1994, vol. 39, 1833-1839
- **N. FURUYA ; T. YAMAZAKI ; M. SHIBATA.** *J. Electroanal. Chem.,* 1997, vol. 431, 39-41